# EUROPEAN PATENT SPECIFICATION

(11) **EP 2 939 710 B1**
(45) Date of publication and mention of the grant of the patent: **17.06.2020**
(21) Application number: 14166486.2
(22) Date of filing: 29.04.2014
(51) Int. Cl.: A61Q 17/04, A61K 8/49, A61K 8/35

(54) **Active Mixtures**
Aktive Mischungen
Mélanges actifs

(43) Date of publication of application: 04.11.2015
(73) Proprietor: Symrise AG, 37603 Holzminden (DE)
(72) Inventor: Johncock, William, 21465 Reinbek (DE)
(74) Representative: Fabry, Bernd

(56) References cited:
- EP-A1- 1 466 892
- EP-A2- 1 213 010
- WO-A1-03/084496
- WO-A1-03/092643
- WO-A1-2005/107692
- WO-A1-2009/020676
- WO-A1-2011/101250
- DE-A1- 10 140 548
- DE-A1- 10 154 111
- DE-A1-102004 038 485
- KATRIN WEINERT ET AL: "Vermeidungvon Textilverfleckung durch den Einsatz wasserlöslicher UVA-Filter", NEUES AUS DERMATOLOGIE, KOSMETIK UND WUNDVERSORGUNG,, no. 29, 1 January 2013 (2013-01-01), pages 17-19, XP009188449,

## Description

### FIELD OF INVENTION

The present invention belongs to the area of cosmetic and pharmaceutical preparations, especially dermatological preparations and their used for the protection of the human skin and human hair against the harmful effects of ultraviolet solar radiation.

### TECHNOLOGICAL BACKGROUND

UV absorbers are compounds which have a pronounced absorption capacity for ultraviolet radiation. They are used in particular as sunscreens in cosmetic and pharmaceutical preparations, especially in dermatological preparations, but also to improve the light fastness of industrial products, such as paints, varnishes, plastics, textiles, polymers such as, for example, polymers and copolymers of mono- and diolefins, polystyrenes, polyurethanes, polyamides, polyesters, polyureas and polycarbonates, packaging materials and rubbers.

UV rays are classified according to wavelength as UVA rays (320-400 nm, UVA-I: 340-400 nm, UVA-II: 320-340 nm) or UVB rays (280-320 nm). UV rays can cause acute and chronic damage to the skin, the type of damage depending on the wavelength of the radiation. For instance, UVB radiation can cause sunburn (erythema) extending to most severe burning of the skin; reduction in enzyme activities, weakening of the immune system, disturbances of the DNA structure and changes in the cell membrane are also known as harmful effects of UVB rays. UVA rays penetrate into deeper layers of the skin where they can accelerate the aging process of the skin. The shorter wave UVA-II radiation additionally contributes to the development of sunburn. Moreover, UVA radiation can trigger phototoxic or photoallergic skin reactions. Very frequent and unprotected irradiation of the skin by sunlight leads to a loss of skin elasticity and to increased development of wrinkles. In extreme cases, pathogenic changes in the skin extending to skin cancer are observed. To attenuate these negative effects of UV radiation, materials which absorb or reflect UV light, generally called UV absorbers, are used in cosmetic, dermatological and pharmacological preparations. The UV absorbers are classified as UVA and UVB absorbers depending on the location of their absorption maxima; if a UV absorber absorbs both UVA and UVB; it is referred to as a UVA/B broadband absorber.

Since 2006 the EU commission (Official Journal of the European Eureopean Union L265/39, September 22, 2006) have recommended that all cosmetic sunscreen formulations should have a UV protection factor which is at least one third of the labelled SPF, in other words an SPF 50+ has to have a UVA protection factor of at least 20, an SPF 50 product has to have a UVA protection factor of 17. Similar regulations are in place in other countries such Brazil and the ASEAN countries. An accepted UVA protection factor efficacy method is described by the Colipa Method for in vitro determination of UVA protection, 2011 or the closely related ISO norm ISO 24443-2012 Determination of sunscreen UVA photoprotection in vitro.

The choice of UV filters along with a maximum amount for each UV filter which can be used in cosmetic and pharmaceutical, especially dermatological preparations is regulated by law in most countries. UVA protection factors of up to 12 can be obtained with the maximum allowed amount of any single UVA filter, so to obtain higher UVA protection factors to comply with regulations, mixtures of UVA filters have to be used.

The number of suitable UVA absorbers is very limited and the most commonly used is 4-tert.-butyl-4'-methoxy-dibenzoylmethane (Butyl methoxydibenzoylmethane, Avobenzone). Avobenzone is currently the UVA filter of choice due to its much lower cost in comparison to other high performing UVA filters. However sunscreen products containing dibenzoylmethane derivatives can leave marks on textiles.

The UVA filters 2,4-bis[{(4-(2-ethylhexyloxy)-2-hydroxy}phenyl]-6-(4-methoxyphenyl)-1,3,5-triazine, (Tinosorb®S), hexyl 2-(4-diethylamino-2-hydroxybenzoyl)benzoate (Uvinul® A Plus) and 2-hydroxy-4-methoxybenzophenone (Benzophenone-3, Oxybenzone, Neo Heliopan® BB), Tris-Biphenyl Triazine (Tinosorb® A2B) are also used to improve the UVA protection of formulations but these cause the staining problem of clothing to be accentuated and these stains are even more difficult to wash out, particularly when mixtures of these UVA filters are used.

The staining problem is recognized by manufactures of sunscreens and some of these put warning statements on the packing alerting the consumer about this. When the words sunscreen staining is googled, over 1 million hits are registered indicating how consumers are concerned about the problem.

### RELEVANT STATE OF THE ART

WO 2003 084496 A1 (SYMRISE) discloses in Example 9 a sunscreen composition comprising sodium phenyl benzimidaole tetraulfonic acid, Avobenzone, 2-ethylhexyl salicylate and diethylhexyl-2,6-naphthalate.

### PROBLEM TO BE SOLVED

Object of the present invention is thus to provide a new composition for cosmetic and pharmaceutical preparations, especially for dermatological preparations, to protect human skin against the harmful effects of UV radiation, without the prior disadvantages mentioned above, in particular without the disadvantage of sunscreen staining.

### DESCRIPTION OF THE INVENTION

What claimed is an aqueous mixture comprising
(a) disodium phenyl benzimidazole tetrasulfonic acid and/or its salts, and
(b) 1-(4-Methoxyphenyl)-3-(4-tert-butylphenyl)propane-1,3-dione (Avobenzone), and
(c) diethylhexyl naphthalate, and
(d) at least one additional UV absorbing substance selected from the group consisting of:
   - 2-ethylhexyl 2-cyano-3,3-diphenylacrylate
   - 2-ethylhexyl p-methoxycinnamate
   - isoamyl p-methoxycinnamate
   - 2,4,6-trianilino(p-carbo-2'-ethylhexyl-1'-oxy)-1,3,5-triazine
   - 3-(4'-methylbenzylidene)-d,l-camphor
   - Benzophenone-4
   - menthyl anthranilate
   - micro fine zinc oxide, and
(e) water
   for use as a medicament for protecting human skin from the harmful effects of ultraviolet radiation of from 280 to 400 nm.

The mixtures are useful in a method of protecting human skin from the harmful effects of ultraviolet radiation of from 280 to 400 nm, comprising applying to human skin an effective amount of a preparation, without causing a staining problem of clothes when formulated into a cosmetic or pharmaceutical composition.

This means that preparations according to the invention are especially useful for making cosmetic and/or pharmaceutical formulations.

### COSMETIC AND PHARMACEUTICAL PREPARATIONS

Cosmetic and pharmaceutical preparations comprising the mixtures according to the present invention may include similar additives, such as for example oil bodies or emulsifiers. Therefore, the border between cosmetic and pharmaceutical preparations is in flow and it should be understood that components cited for one application are recommended for the other *mutatis-mutandis* without literal repetition.

### SURFACTANTS

Preferred embodiments of the cosmetic and pharmaceutical, especially dermatological preparations may also comprise anionic, cationic, nonionic and/or amphoteric surfactants (included in the term surfactant is the term emulsifier). Surfactants are amphiphilic substances which can dissolve or disperse organic, nonpolar substances in water. In this context, the hydrophilic components of a surfactant molecule are usually polar functional groups, for example -COO⁻, -OSO₃²⁻, -SO₃⁻, while the hydrophobic parts as a rule are nonpolar hydrocarbon radicals. Surfactants are in general classified according to the nature and charge of the hydrophilic molecular moiety. A distinction can be made between four groups here:
- anionic surfactants,
- cationic surfactants,
- amphoteric surfactants and
- nonionic surfactants.

**Anionic surfactants** as a rule contain carboxylate, sulphate or sulphonate groups as functional groups. In aqueous solution, they form negatively charged organic ions in an acid or neutral medium. Cationic surfactants are almost exclusively characterized by the presence of a quaternary ammonium group. In aqueous solution, they form positively charged organic ions in an acid or neutral medium. Amphoteric surfactants contain both anionic and cationic groups and accordingly behave like anionic or cationic surfactants in aqueous solution, depending on the pH. In a strongly acid medium they have a positive charge, and in an alkaline medium a negative charge. On the other hand, they are zwitterionic in the neutral pH range. Polyether and polysaccharide chains are typical of nonionic surfactants. Nonionic surfactants do not form ions in an aqueous medium. Specificall useful are:
- acylamino acids (and salts thereof), such as:
- acyl glutamates, for example sodium acyl glutamate, di-TEA-palmitoyl aspartate and sodium caprylic/capric glutamate,
- acyl peptides, for example palmitoyl hydrolysed milk protein, sodium cocoyl hydrolysed soya protein and sodium/potassium cocoyl hydrolysed collagen,
- sarcosinates, for example myristoyl sarcosine, TEA-lauroyl sarcosinate, sodium lauroyl sarcosinate and sodium cocoyl sarcosinate,
- taurates, for example sodium lauroyl taurate and sodium methylcocoyl ta urate,
- acyl lactylates, lauroyl lactylate, caproyl lactylate
- alaninates
- carboxylic acids and derivatives, such as for example: TEA stearate, Glyceryl stearates, PEG glyceryl stearates, lauric acid, aluminium stearate, magnesium alkanolate and zinc undecylenate,
- ester-carboxylic acids, for example: calcium stearoyl lactylate, laureth-6 citrate and sodium PEG-4 lauramide carboxylate, glyceryl stearates, glyceryl-oleylstearates, glyceryl citrates, glyceryl oleyl citrates,
- ether-carboxylic acids, for example sodium laureth-13 carboxylate and sodium PEG-6 cocamide carboxylate,
- Glucoside esters, such as for example:
- cetearyl glucoside, lauryl glucoside
- phosphoric acid esters and salts, such as, for example: cetyl phosphate (mono, di cetyl and their mixtures), Potassium cetyl phosphate, (mono, di cetyl and their mixtures), DEA cetyl phosphate (mono, di cetyl and their mixtures), DEA -oleth-10 phosphate and dilaureth-4 phosphate,
- sulphonic acids and salts, such as acyl isethionates, e.g. sodium/ammonium cocoyl isethionate, alkylarylsulphonates,
- alkylsulphonates, for example sodium coco-monoglyceride sulphate, sodium C12-14 olefinsulphonate, sodium lauryl sulphoacetate and magnesium PEG-3 cocamide sulphate,
- sulphosuccinates, for example dioctyl sodium sulphosuccinate, disodium laureth-sulphosuccinate, disodium laurylsulphosuccinate and disodium undecylenamido-MEA-sulphosuccinate and
- sulphuric acid esters, such as: alkyl ether sulphate, for example sodium, ammonium, magnesium, MIPA, TIPA laureth sulphate, sodium myreth sulphate and sodium C12-13 pareth sulphate,
- alkyl sulphates, for example sodium, ammonium and TEA lauryl sulphate.

**Cationic surfactants** which are advantageously used are
- alkylamines,
- alkylimidazoles,
- ethoxylated amines,
- quaternary surfactants,
- RNH₂CH₂CH₂COO⁻ (at pH=7)
- RNHCH₂CH₂COO- B⁺ (at pH=12) B⁺ = any desired cation, e.g. Na⁺ and
- esterquats.

Quaternary surfactants contain at least one N atom which is covalently bonded to 4 alkyl or aryl groups. This leads to a positive charge, independently of the pH. Alkylbetaine, alkylamidopropylbetaine and alkylamidopropylhydroxysulphaine are advantageous. The cationic surfactants used can further preferably be chosen from the group consisting of quaternary ammonium compounds, in particular benzyltrialkylammonium chlorides or bromides, such as, for example, benzyldimethylstearylammonium chloride, and also alkyltrialkylammonium salts, for example cetyltrimethylammonium chloride or bromide, alkyldimethylhydroxyethylammonium chlorides or bromides, dialkyldimethylammonium chlorides or bromides, alkylamideethyltrimethylammonium ether sulphates, alkylpyridinium salts, for example lauryl- or cetylpyridinium chloride, imidazoline derivatives and compounds having a cationic character, such as amine oxides, for example alkyldimethylamine oxides or alkylaminoethyldimethylamine oxides. Cetyltrimethyl-ammonium salts in particular are advantageously used.

**Amphoteric surfactants** which are advantageously to be used are:
- acyl/dialkylethylenediamine, for example sodium acylamphoacetate, disodium acylamphodipropionate, disodium alkylamphodiacetate, sodium acylamphohydroxypropylsulphonate, disodium acylamphodiacetate and sodium acylamphopropionate,
- N-alkylamino acids, for example aminopropyl alkylglutamide, alkylaminopropionic acid, sodium alkylimidodipropionate and lauroamphocarboxyglycinate.
- acylamphohydroxypropylsulphonate, disodium acylamphodiacetate and sodium acylamphopropionate,
- N-alkylamino acids, for example aminopropyl alkylglutamide, alkylaminopropionic acid, sodium alkylimidodipropionate and lauroamphocarboxyglycinate.

**Nonionic surfactants** which are advantageously used are
- alcohols,
- alkanolamides, such as cocamides MEA/DEA/MIPA,
- amine oxides, such as cocoamidopropylamine oxide,
- ethers, for example ethoxylated/propoxylated alcohols, ethoxylated/propoxylated esters, ethoxylated/propoxylated glycerol esters, ethoxylated/propoxylated cholesterols, ethoxylated/propoxylated triglyceride esters, ethoxylated/propoxylated lanolin, ethoxylated/propoxylated polysiloxanes, propoxylated POE ethers and alkyl polyglycosides, such as lauryl glucoside, decyl glucoside and coco-glycoside.
- sucrose esters, sucrose ethers
- polyglycerol esters, diglycerol esters, monoglycerol esters polyglyceryl-2 dipolyhydroxystearate (Dehymuls® PGPH), polyglyceryl-3 diisostearate (Lameform® TGI), polyglyceryl-4 isostearate (Isolan® GI 34), polyglyceryl-3 oleate, diisostearyl polyglyceryl-3 diisostearate (Isolan® PDI), polyglyceryl-3 methylglucose distearate (Tego Care® 450), polyglyceryl-3 beeswax (Cera Bellina®), polyglyceryl-4 caprate (polyglycerol caprate T2010/90), polyglyceryl-3 cetyl ether (Chimexane® NL), polyglyceryl-3 distearate (Cremophor® GS 32), polyglyceryl-2 stearate (Hostacerin® DGMS) and polyglyceryl polyricineoleate (Admul® WOL 1403), and mixtures thereof.
- methylglucose esters, esters of hydroxy acids

The use of a combination of anionic and/or amphoteric surfactants with one or more nonionic surfactants is further advantageous.

### OIL BODIES

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic ca rboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 ca r-bon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### EMULSIFIERS

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

**Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

**Sorbitan esters.** Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

**Polyglycerol esters.** Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan ® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) and Polyglyceryl Polyricinoleate (Admul® WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

**Anionic emulsifiers.** Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

**Amphoteric emulsifiers.** Other suitable emulsifiers are amphboteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example coco-acylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### SUPERFATTING AGENTS AND CONSISTENCY FACTORS

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### THICKENING AGENTS AND RHEOLOGY ADDITIVES

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### POLYMERS

Suitable polymers for the cosmetic and/or pharmaceutical, especially dermatologically preparations may advantageously also comprise the use of polymers to imrove the spreadibility of the formulation upon the skin or hair, or improve the water and or sweat and or rub-off resistancy of the formula and to improve the protection factor of the formulation. Examples of such polymers are : VP/Eicosene copolymers sold under the trade name of Antaron V-220 by International Speciality Products, VP/Hexadecene copolymer sold under the trade names Antaron V-216 and Antaron V-516 by International Speciality Products, Tricontanyl PVP sold under the trade name of Antaron WP-660 by International Speciality Products, Isohexadecane and Ethylene/Propylene/Styrene copolymer and Butylene/Styrene copolymer sold under the trade names of Versagel MC and MD by Penreco, Hydrogenated polyisobutene and and Ethylene/Propylene/Styrene copolymer and Butylene/Styrene copoly-mer sold under the trade mane of Versagel ME by Penreco, Acrylates/Octylacrylamide Coploymers sold under the trade name of Dermacryl 79, Dermacryl AQF and Dermacryl LT by National Starch, Polyurethanes such as PPG-17/IPDI/DMPA copolymer sold under the trade name of Avalure UR 450 & 525 sold by Noveon, Polyurethanes-2 and -4 sold under the trade names Avalure UR-405, -410, - 425, -430 and - 445 525 sold by Noveon, Polyurethane 5 and Butyl Acetate and isopropyl alcohol sold under the trade name Avalure UR - 510 and - 525 sold by Noveon, Polyurethanes -1 and - 6 sold under the trade name of Luviset PUR by BASF, Hydrogenated Dimer Dilinoleyl/Dimethylcarbonate Copolymer sold under the trade name of Cosmedia DC by Cognis.

Of course, as one well versed in the art of cosmetic, dermatological and pharmacological preparations knows, this is not an exhaustive list and other suitable polymers not listed here may be used. Examples of such polymers may be found in the latest edition of the CTFA's International Cosmetic Ingredient Dictionary

The amount of polymers used to obtain the desired effect in the formulation range from 0.10% to 5.0% by weight of the fomulation and especially in the range from 0.25% to 3.0% by weight of the formulation.

### PEARLISING WAXES

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### SILICONES

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates.

### WAXES AND STABILIZERS

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

### COOLING AGENTS

Cosmetic and/or pharmaceutical, especially dermatologically active may also comprise substances having a cooling action. Individual active cooling compounds which are preferred for use are listed below. The skilled person is able to supplement the following list with a large number of further active cooling compounds; the active cooling compounds listed can also be employed in combination with one another: I-menthol, d-menthol, racemic menthol, menthone glycerol acetal (trade name: Frescolat®MGA), menthyl lactate (trade name: Frescolat®ML, menthyl lactate is preferably I-menthyl lactate, in particular I-menthyl I-lactate), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxyethyl menthyl carbonate, 2-hydroxypropyl menthyl carbonate, N-acetylglycine menthyl ester, isopulegol, menthyl hydroxycarboxylic acid esters (e.g. menthyl 3-hydroxybutyrate), monomenthyl succinate, 2-mercaptocyclodecanone, menthyl 2-pyrrolidin-5-onecarboxylate, 2,3-dihydroxy-p-menthane, 3,3,5-trimethylcyclo-hexanone glycerol ketal, 3-menthyl 3,6-di- and -trioxaalkanoates, 3-menthyl methoxyacetate, icilin.

Preferred active cooling compounds are: I-menthol, d-menthol, racemic menthol, menthone glycerol acetal (trade name: Frescolat®MGA), menthyl lactate (preferably I-menthyl lactate, in particular I-menthyl I-lactate, trade name: Frescolat®ML), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxtethyl menthyl carbonate, 2-hydroxypropyl menthyl carbonate, isopulegol.

Particularly preferred active cooling compounds are: I-menthol, racemic menthol, menthone glycerol acetal (trade name: Frescolat®MGA), menthyl lactate (preferably I-menthyl lactate, in particular I-menthyl I-lactate, trade name: Frescolat®ML), 3-menthoxypropane-1,2-diol, 2-hydroxyethyl menthyl carbonate, 2-hydroxypropyl menthyl carbonate.

Very particularly preferred active cooling compounds are: I-menthol, menthone glycerol acetal (trade name: Frescolat®MGA), menthyl lactate (preferably I-menthyl lactate, in particular I-menthyl I-lactate, trade name: Frescolat®ML).

The use concentration of the active cooling compounds to be employed is, depending on the substance, preferably in the concentration range from 0.01% to 20% by weight, and more preferably in the concentration range from 0.1% to 5% by weight, based on the total weight of the completed (ready-to-use) cosmetic, dermatological and pharmacological preparations.

### ANTI-MICROBIAL AGENTS

Suitable anti-microbial agents are, in principle, all substances effective against Grampositive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### ENZYME INHIBITORS

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### ODOUR ABSORBERS AND ANTIPERSPIRANT ACTIVE AGENTS

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### FILM FORMERS AND ANTI-DANDRUFF AGENTS

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethylpentyl)-2-(1H)-pyridinone monoethanolamine salt), Baypival® (Climbazole), Ketoconazol® (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon® UD (protein/undecylenic acid condensate), zinc pyrithione, aluminium pyrithione and magnesium pyrithione/dipyrithione magnesium sulfate.

### CARRIERS AND HYDROTROPES

Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations may be produced using preservatives or solubilizers. Other preferred liquid carrier substances, which may be a component of a preparation are selected from the group consisting of oils such as vegetable oil, neutral oil and mineral oil.

Preferred solid carrier materials, which may be a component of a preparation are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### PREERVATIVES

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

Preferred embodiments of the cosmetic and/or pharmaceutical, especially dermatologically active preparations may in numerous cases advantageously comprise the following preservatives:

Preservatives which are preferably chosen here are those such as benzoic acid, its esters and salts, propionic acid and its salts, salicylic acid and its salts, 2,4 hexadienoic acid (sorbic acid) and its salts, formaldehyde and paraformaldehyde, 2-hydroxybiphenyl ether and its salts, 2-zincsulphidopyridine N-oxide, inorganic sulphites and bisulphites, sodium iodate, chlorobutanolum, 4 ethylmercuryl(II)-5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury(II)-thiosalicylic acid, phenylmercury and its salts, 10-undecylenic acid and its salts, 5-amino-1,3-bis(2-ethylhexyl)-5-methylhexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)urea, 4 chloro-m-cresol, 2,4,4'-trichloro-2'-hydroxydiphenyl ether, 4-chloro-3,5-dimethylphenol, 1,1'-methylene-bis(3-(1-hydroxymethyl-2,4-dioximidazolidin-5 yl)urea), poly(hexamethylene diguanide) hydrochloride, 2-phenoxyethanol, hexamethylenetetramine, 1-(3-chloroallyl)-3,5,7-triaza-1-azoniaadamantane chloride, 1-(4-chlorophenoxy)-1-(1H-imidazol-1-yl)-3,3-dimethyl-2-butanone, 1,3 bis-(hydroxymethyl)-5,5-dimethyl-2,4-imidazolidinedione, benzyl alcohol, octopirox, 1,2-dibromo-2,4-dicyanobutane, 2,2'-methylenebis(6-bromo-4-chloro¬phenol), bromochlorophene, mixture of 5-chloro-2-methyl-3(2H)-isothiazolinone and 2-methyl-3(2H)isothiazolinone with magnesium chloride and magnesium nitrate, 2-benzyl-4-chlorophenol, 2-chloroacetamide, chlorhexidine, chlorhexidine acetate, chlorhexidine gluconate, chlorhexidine hydrochloride, 1-phenoxypropan-2-ol, N-alkyl(C12-C22)trimethylammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidino-phenoxy)-n-hexane and its salts, glutaraldehyde, 5-ethyl-1-aza-3,7-dioxabicyclo-[3.3.0]octane, 3-(4-chlorophenoxy)-1,2-propanediol, hyamines, alkyl-(C8-C18)-dimethylbenzylammonium chloride, alkyl-(C8-C18)-dimethylbenzylammonium bromide, alkyl-(C8-C18)-dimethylbenzyl-ammonium saccharinate, benzyl hemiformal, 3-iodo-2-propynyl butylcarbamate, sodium hydroxymethylaminoacetate or sodium hydroxymethylaminoacetate.

In various cases it may also be advantageous to employ substances which are chiefly em-ployed for inhibition of the growth of undesirable microorganisms on or in animal organisms in cosmetic and/or pharmaceutical, especially dermatologically active, preparations. In this respect, in addition to conventional preservatives, further active compounds which are worth mentioning, in addition to the large group of conventional antibiotics, are, in particular, the products relevant for cosmetics, such as triclosan, climbazol, octoxyglycerol, octopirox (1-hydroxy-4-methyl-6-(2,4,4-trimethylpentyl)-2(1H)-pyridone, 2 aminoethanol), chitosan, farnesol, glycerol monolaurate or combinations of the substances mentioned, which are employed, inter alia, against underarm odour, foot odour or dandruff formation.

### PERFUME OILS AND FRAGRANCES

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, 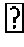 -isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### DYES

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

### ANTI-IRRITATION AGENTS

An important group of co-actives encompass anti-irritant agents such as for example steroidal anti-inflammatory substances of the corticosteroid type, such as e.g. hydrocortisone, hydrocortisone derivatives, such as hydrocortisone 17-butyrate, dexamethasone, dexamethasone phosphate, methylprednisolone or cortisone; non-steroidal anti-inflammatories like oxicams, such as piroxicam or tenoxicam; salicylates, such as aspirin, Disalcid, Solprin or fendosal; acetic acid derivatives, such as diclofenac, fenclofenac, indomethacin, sulindac, tolmetin or clindanac; fenamates, such as mefenamic, meclofenamic, flufenamic or niflumic; propionic acid derivatives, such as ibuprofen, naproxen or benoxaprofen, or pyrazoles, such as phenylbutazone, oxyphenylbutazone, febrazone or azapropazone. Alternatively, natural anti-inflammatory substances or reddening- and/or itching-alleviating substances can be employed. Plant extracts, specific highly active plant extract fractions and highly pure active substances isolated from plant extracts, can be employed like extracts, fractions and active substances from aloe vera, Commiphora species, Rubia species, Rubus species, willow, rose-bay, willowherb, oats, calendula, arnica, St. John's wort, honeysuckle, ginger, chamomile, rosemary, sage, melissa, Passiflora incarnata, Sophora japonica, witch hazel, Pueraria, Dianthus or Echinacea, as well as pure substances, such as, inter alia, bisabolol, apigenin, apigenin-7-glucoside, rosmarinic acid, boswellic acid, phytosterols, glycyrrhizic acid, glabridin, licochalcone A, [6]-paradol, and anthranilic acid amides, such as, in particular, avenanthramides or dianthramides, are particularly preferred. The total amount of anti-irritants in a formulation is preferably in the range of from 0.0001 to 20 wt.%, preferably from 0.0001 to 10 wt.%, in particular from 0.001 to 5 wt.%, based on the total weight of the formulation or product, respectively.

Particular useful co-actives are selected from the group consisting of anti-mycotica and pain relief agents, and more particularly the group consisting of erythromycin, dimeti n-dene, betamethasone, ibuprofen, ketoprofene, diclofenac, metronidazole, acyclovir, imiquimod, terbinafine, docosanol, cyclopyroxolamine, and their mixtures:

**Erythromycin** is a macrolide antibiotic that has an antimicrobial spectrum similar to or slightly wider than that of penicillin, and is often used for people who have an allergy to penicillin.

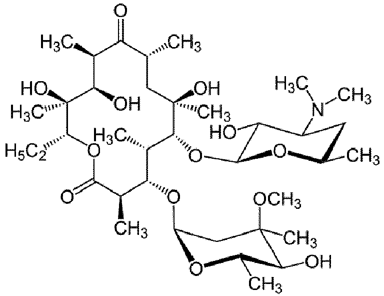

Recent studies have also shown that it can be used as a mild anti-depressant. For respiratory tract infections, it has better coverage of atypical organisms, including *Myco-plasma* and legionellosis. It was first marketed by Eli Lilly and Company, and it is today commonly known as EES (erythromycin ethylsuccinate, an ester prodrug that is commonly administered). In structure, this macrocyclic compound contains a 14-membered lactone ring with ten asymmetric centres and two sugars (L-cladinose and D-desosamine), making it a compound very difficult to produce via synthetic methods. Erythromycin is produced from a strain of the actinomycete *Saccharopolyspora erythraea* (see US 2,653,899 - Eli Lily).

**Dimetindene,** also known as **Fenistil** (RS-dimethyl(2-(3-[pyridin-2-yl)ethyl]-1H-inden-2-yl)ethyl)amine) is an antihistamine/anticholinergic used orally and locally as an antipruritic.

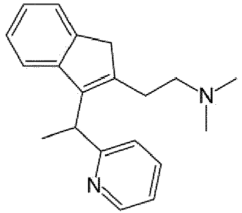

**Betamethasone** (8S,9R,10S.11S,13S,14S,16S,17R)-9-fluoro-11,17-(2-hydroxyacetyl)-10, 13,16-trimethyl-6,7,8,9,10,11,12,13,14,15,16,17-dodecahydro-3H-cyclopenta(alpha)-phenanthren-3-one) is a potent glucocorticoid steroid with anti-inflammatory and immunosuppressive properties.

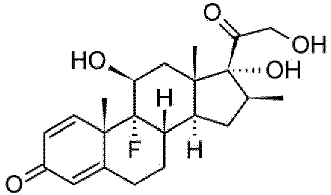

Unlike other drugs with these effects, betamethasone does not cause water retention. It is applied as a topical cream, ointment, foam, lotion or gel to treat itching. Betamethasone sodium phosphate is sometimes prescribed as an intramuscular injection (I.M) for itching from various ailments, including allergic reactions to poison ivy and similar plants (see US 3,053,865 - Merck).

**Ibuprofen** (RS)-2-(4-(2-methylpropyl)phenyl)propanoic acid) from the nomenclature iso-butyl-propanoic-phenolic acid) is a non-steroidal anti-inflammatory drug (NSAID) used for relief of symptoms of arthritis, fever, as an analgesic (pain reliever), especially where there is an inflammatory component, and dysmenorrhea.

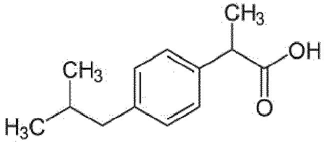

Ibuprofen is known to have an antiplatelet effect, though it is relatively mild and somewhat short-lived when compared with aspirin or other better-known antiplatelet drugs. In general, ibuprofen also acts as a vasoconstrictor, having been shown to constrict coronary arteries and some other blood vessels mainly because it inhibits the vasodilating prostacyclin produced by cyclooxygenase 2 enzymes. Ibuprofen was derived from propanoic acid by the research arm of Boots Group during the 1960s and was patented in 1961. Originally marketed as Brufen, ibuprofen is available under a variety of popular trademarks, including Motrin, Nurofen, Advil, and Nuprin (see US 3,385,886 - Boots).

**Ketoprofen** (RS)2-(3-benzoylphenyl)-propionic acid is another one of the propionic acid class of non-steroidal anti-inflammatory drug (NSAID) with analgesic and antipyretic effects.

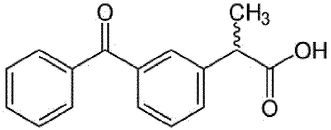

It acts by inhibiting the body's production of prostaglandins (see US 3,641,127 - Rhone-Poulenc).

**Diclofenac** is also a non-steroidal anti-inflammatory drug (NSAID) taken to reduce inflammation and as an analgesic reducing pain in certain conditions.

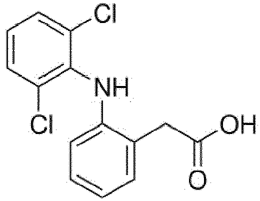

The name is derived from its chemical name: 2-(2,6-dichloranilino) phenylacetic acid. In the United Kingdom, India, Brazil and the United States, it may be supplied as either the sodium or potassium salt, in China most often as the sodium salt, while in some other cou n-tries only as the potassium salt. Diclofenac is available as a generic drug in a number of formulations. Over-the-counter (OTC) use is approved in some countries for minor aches and pains and fever associated with common infections (see US 3,558,690 - Ciba-Geigy).

**Metronidazole** (2-(2-methyl-5-nitro-1H-imidazol-1-yl)ethanol) is a nitroimidazole antibiotic medication used particularly for anaerobic bacteria and protozoa.

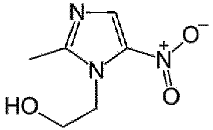

Metronidazole is an antibiotic, amebicide, and antiprotozoal. It is the drug of choice for first episodes of mild-to-moderate Clostridium difficile infection. It is marketed in the U.S.A. by Pfizer and globally by Sanofiunder the trade name Flagyl, in Pakistan and Bangladesh also as Nidagyl by Star Laboratories, and in Thailand, as Mepagyl by Thai Nakhorn Patana. It is also marketed in UK by Milpharm Limited and Almus Pharmaceuticals. Metronidazole was developed in 1960. Metronidazole is used also as a gel preparation in the treatment of the dermatological conditions such as rosaceae and fungating tumours (see US 2,944,061-Rhone Poulenc).
(VIII) **Acyclovir** or acyclovir (USAN, former BAN), chemical name acycloguanosine (2-Amino-1,9-dihydro-9-((2-hydroxyethoxy)methyl)-6H-Purin-6-one), abbreviated as ACV is a guanosine analogue antiviral drug, marketed under trade names such as *Cyclovir, Herpex, Acivir, Acivirax, Zovirax,* and *Xovir.* The solid active agent has a solubility in water (20° dH) at 20 °C of less than 5 g/L.

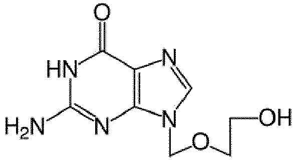

One of the most commonly used antiviral drugs; it is primarily used for the treatment of herpes simplex virus infections, as well as in the treatment of varicella zoster (chickenpox) and herpes zoster (shingles); see also US 4,199,574 (Wellcome).

**Imiquimod** (3-(2-methylpropyl)-3,5,8-triazatricyclo[7.4.0.0.^{2,6}]trideca-1(9),2(6),4,7,10, 12- hexaen-7-amine, INN) is a prescription medication that acts as an immune response modifier.

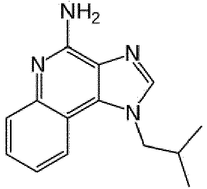

It is marketed by Meda AB, Graceway Pharmaceuticals and iNova Pharmaceuticals under the trade names Aldara and Zyclara, and by Mochida as Beselna. It is also referred to as R-837 (see US 4,689,338 - Riker).

**Terbinafine,** more particularly terbinafine hydrochloride [(2E)-6,6-dimethylhept-2-en-4-yn-1-yl](methyl)(naphthalen-1-ylmethyl)amine) is a synthetic allylamine antifungal from Novartis. It is highly lipophilic in nature and tends to accumulate in skin, nails, and fatty tissues.

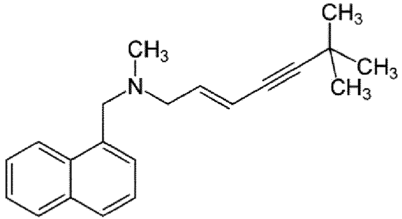

It is sold by the name Lamisil in Argentina, Australia, Belgium, Brazil, Canada, Chile, Egypt, Finland, France, Germany, Greece, Hungary, Iceland, Ireland, Israel, Mexico, Pakistan, Peru, New Zealand, Norway, Romania, Russia, Slovenia, South Africa, Sweden, United Kingdom, United States and Venezuela, also sold under the name Corbinal andTerbisil in Turkey and under the name "undofen cream" in Poland. As a generic it is sold under the name Zabel in Australia. It is also available as a generic medication in the United States, United Kingdom, Belgium, Switzerland and Brazil. In India, Terbinafine hydrochloride is available in topical form under the brand name Sebifin (Ranbaxy Labs), Zimig (GSK Pharma) and mycoCeaze (Progres Laboratories).MycoVa, developed by Apricus Biosciences, is a topical nail solution of terbinafine and DDAIP which has completed three Phase III studies for the treatment of onychomycosis (see US 4,755,534 - Sandoz)

**Docosanol,** also known as behenyl alcohol, is a saturated fatty alcohol used traditionally as an emollient, emulsifier, and thickener in cosmetics, nutritional supplement (as an individual entity and also as a constituent of policosanol), and more recently, in a Food and Drug Administration (FDA) approved pharmaceutical, Abreva, approved as an antiviral agent for reducing the duration of cold sores caused by the herpes simplex virus.

**Ciclopiroxolamine** (6-cyclohexyl-1-hydroxy-4-methylpyridin-2(1H)-one) also called Batrafen, Loprox, Mycoster, Penlac and Stieprox, is a synthetic antifungal agent for topical dermatologic treatment of superficial mycoses.

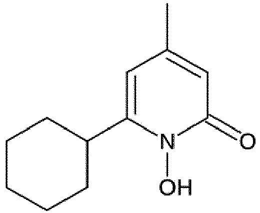

It is most useful against Tinea versicolor (see US 3,883,545 - Marck).

### ANTI-CELLULITE AGENTS

Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

### FAT ENHANCING AGENTS

The formulations may also comprise one or more fat enhancing and/or adipogenic agents as well as agents enhancing or boosting the activity of fat enhancing agents. A fat enhancing agent is for example hydroxymethoxyphenyl propylmethylmethoxybenzofuran (trade name: Sym3D™).

### HAIR GROWTH ACTIVATORS OR INHIBITORS

The formulations may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, anti-androgenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example betasitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, licorice, grape, apple, barley or hops or/nd hydrolysates from rice or wheat.

Alternatively, the formulations may comprise one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

### SOLUTES

The formulations may also comprise one or more compatible solutes. Preferred compatible solutes are such as described in WO 01/76572, particularly dimyo-inositol phosphate (DIP), diglycerin phospate (DGP), di-myo-inositol phosphate (DIP), cyclic 2,3 diphosphoglycerate (cDPG), 1,1-di-glycerol phosphate (DGP), beta-mannosyl glycerate (firoin), beta-mannosyl glyceramide (firoin-A) and di-mannosyl-di-inositol phosphate (DMIP) and ectoine and ectoine-derivatives, as described in EP 0 553 884, EP 0 671 161 and WO 94/15923, in particular ((S)-1,4,5,6-tetrahydro-2-methyl-4-pyrimidinecarboxylic acid) and hydroxyectoine ((S,S)-1,4,5,6-tetrahydro-5-hydroxy-2-methyl-4-pyrimidinecarboxylic acid).

Preferably, the total amount of compatible solutes is in the range of from 0.05 to 10 wt.-%, preferably from 0.1 to 5 wt.-%, based on the total weight of the formulation or product.

### SOLVENTS

The formulations may contain such as for example aliphatic alcohols or 1,2-alkandiols or of course simply water. Suitable 1,2-alkandiols encompass 1,2-butadiol, 1,2-pentandiol, 1,2-hexandiol, 1,2-heptanddiol, 1,2-octandiol, 1,2-nonandiol, 1,2-decandiol, 1,2-undecandiol, 1,2,dodecandiol and their mixtures. The preferred 1,2-alkandiol is 1,2-pentandiol. Suitable aliphatic alcohols are selected from the group consisting of ethanol, n-propanol, isopropylalcohol, the isomeric butanols and their mixtures. The preferred species is ethanol, in particular with a purity of at least 95 %.

### PIGMENTS

Cosmetic and/or pharmaceutical preparations advantageously, but not obligatorily, comprise inorganic pigments based on finely disperse metal oxides and/or other metal compounds which are insoluble or sparingly soluble in water, in particular the oxides of titanium (TiO₂), zinc (ZnO), iron (e.g. Fe₂O₃), zirconium (ZrO₂), silicon (SiO₂), manganese (e.g. MnO), aluminum (A1₂O₃), cerium (e.g. Ce₂O₃), mixed oxides of the corresponding metals, and mixtures of such oxides. These pigments are X-ray-amorphous or non-X-ray-amorphous. X-ray-amorphous oxide pigments are metal oxides or semi-metal oxides which reveal no or no recognizable crystalline structure in X-ray diffraction experiments. Such pigments are often obtainable by flame reaction, for example by reacting a metal or semi-metal halide with hydrogen and air (or pure oxygen) in a flame.

In cosmetic and/or pharmaceutical preparations, X-ray-amorphous oxide pigments are used as thickeners and thixotropic agents, flow auxiliaries for emulsion and dispersion stabilization and as carrier substance (for example for increasing the volume of finely divided powders). X-ray-amorphous oxide pigments which are known and often used in cosmetic or dermatological galenics are, for example, high-purity silicon oxide. Preference is given to high-purity, X-ray-amorphous silicon dioxide pigments with a particle size in the range from 5 to 40 nm and an active surface area (BET) in the range from 50 to 400 m²/g, preferably 150 to 300 m²/g, where the particles are to be regarded as spherical particles of very uniform dimension. Macroscopically, the silicon dioxide pigments are recognizable as loose, white powders. Silicon dioxide pigments are sold commercially under the name Aerosil (CAS-No. 7631-85-9) or Carb-O-Sil.

Advantageous Aerosil ® grades are, for example, Aerosil®0X50, Aerosil ®130, Aerosil ®150, Aerosil ®200, Aerosil ®300, Aerosil ®380, Aerosil ®MQX 80, Aerosil ® MOX 170, Aerosil ®COK 84, Aerosil ® R 202, Aerosil ®R 805, Aerosil ®R 812, Aerosil ®R 972, Aerosil ®R 974, Aerosil ®R976.

Said cosmetic and pharmacological preparations, preferably dermatological preparations may comprise 0.1 to 20% by weight, advantageously 0.5 to 10% by weight, more preferably 1 to 5% by weight, of X-ray-amorphous oxide pigments.

The non-X-ray-amorphous inorganic pigments are advantageously in hydrophobic form, i.e. have been surface-treated to repel water. This surface treatment may involve providing the pigments with a thin hydrophobic layer by processes known per se. Such a process involves, for example, producing the hydrophobic surface layer by a reaction according to

n TiO₂ + m (RO)₃Si-R' → n TiO₂ (surf.)

where n and m are stoichiometric parameters to be used as desired, and R and R' are the desired organic radicals. Hydrophobic pigments prepared analogously to DE-A 33 14 742, for example, are advantageous.

The total amount of inorganic pigments, in particular hydrophobic inorganic micro pigments, in the finished cosmetic and pharmacological preparations, particularly in dermatological preparations is advantageously chosen from the range from 0.1 to 30% by weight, preferably 0.1 to 10.0% by weight, preferably 0.5 to 6.0% by weight, based on the total weight of the preparations.

### SKIN LIGHTENING INGREDIENTS

The formulations may also include skin lightening agents selected from the group consisting of kojic acid (5-hydroxy-2-hydroxymethyl-4-pyranone), kojic acid derivatives such as for example kojic dipalmitate, arbutin, ascorbic acid, ascorbic acid derivatives, hydroquinone, hydroquinone derivatives, styryl resorcinol derivatives (e.g. 4-(1-phenylethyl)1,3-benzenediol), molecules containing sulphur, such as glutathione or cysteine for example, alpha-hydroxy acids (e.g. citric acid, lactic acid, malic acid) and their derivatives, N-acetyltyrosine and derivatives, undecenoylphenylalanine, gluconic acid, chromone derivatives such as aloesin, flavonoids, thymol derivatives, 1-aminoethylphosphinic acid, thiourea derivatives, ellagic acid, nicotinamide, zinc salts such as zinc chloride or zinc gluconate for example, thujaplicin and derivatives, triterpenes such as maslic acid, sterols such as ergosterol, benzofuranones such as senkyunolide, vinyl- and ethylguaiacol, dionic acids such as octodecenedionic acid and azelaic acid, nitrogen oxide synthesis inhibitors such as L-nitroarginine and its derivatives, 2,7-dinitroindazole or thiocitrulline, metal chelators (e.g. alpha-hydroxy fatty acids, palmitic acid, phytic acid, lactoferrin, humic acid, gallic acid, bile extracts, bilirubin, biliverdin), retinoids, soja milk, soya extract, serine protease inhibitors or lipoic acid or other synthetic or natural active compounds for skin and hair lightening, these compounds also being used in the form of an extract from plants, such as bearberry extract, rice extract, papaya extract, liquorice root extract or constituents concentrated from these, such as glabridin or licochalcone A, Artocarpus extract, extract from Rumex and Ramulus species, extracts from pine species (Pinus) and extracts from Vitis species or stilbene derivatives concentrated from these, extract from saxifraga, mulberry, Scutelleria and/or grapes.

### ANTIOXIDANTS

An additional content of antioxidants in the cosmetic and pharmacological preparations, preferably dermatological preparations is generally preferred. Favorable antioxidants which can be used are all antioxidants customary or suitable for the cosmetic and pharm a-cological preparations, preferably dermatological preparations. The antioxidants are advantageously chosen from the group of amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine sulfoximines, homocysteine sulfoximine, buthionine sulfones, penta-, hexa-, heptathionine sulfoximine) in very low tolerated doses (e.g. pmol to µmοl/kg), and also (metal) chelating agents (e.g. α-hydroxy fatty acids, palmitic acid, phytic acid, lactoferrin), α-hydroxy acids (e.g. citric acid, lactic acid, maleic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of benzoin resin, rutinic acid and derivatives thereof, α-glycosylrutin, ferulic acid, furfurylideneglucitol, carnosine, butylhydroxy-toluene, butylhydroxyanisol, nordihydroguaiacic acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids), derivatives of acetophenone such as Hydroxyacetophenone and its blends with Phenoxyethanol and/or, pentane 1,2 diol and/or hexane 1,2 diol and/or caprylyl 1,2 diol, are suitable.

The amount of the above-mentioned antioxidants (one or more compounds) in the preparations is preferably 0.001 to 30% by weight, more preferably 0.05 to 20% by weight, and most preferably 1 to 10% by weight, based on the total weight of the preparation.

### VITAMINS

Preferred embodiments of the cosmetic and pharmacological preparations, preferably dermatological preparations may advantageously also comprise vitamins and vitamin precursors, it being possible for all the vitamins and vitamin precursors which are suitable or usual for cosmetic and pharmacological preparations, especially dermatological preparations to be used. Those worth mentioning here are, in particular, vitamins and vitamin precursors, such as tocopherols, vitamin A, niacin acid and niacinamide, further vitamins of the B complex, in particular biotin, and vitamin C and panthenol and derivatives thereof, in particular the esters and ethers of panthenol, and cationically derivatized panthenols, such as panthenol triacetate, panthenol monoethyl ether and the monoacetate thereof and cationic panthenol derivatives. If vitamin E and/or derivatives thereof represent the antioxidant(s), it is advantageous to choose their respective concentrations from the range from 0.001 to 10% by weight, based on the total weight of the formulation. If vitamin A or vitamin A derivatives, or carotenes or derivatives thereof represent the antioxidant(s), it is advantageous to choose their respective concentrations from the range from 0.001 to 10% by weight, based on the total weight of the formulation.

### LIPIDS

Preferred embodiments of the cosmetic and pharmacological preparations, especially dermatological preparations may also comprise lipids chosen from the following group of substances:
(i) linear or branched saturated paraffins (mineral oils) having 15 or more C atoms, in particular having 18 to 45 C atoms;
(ii) esters having 12 or more C atoms of linear or branched fatty acids having 6 to 30 C atoms and linear or branched, saturated or unsaturated mono-, di- or triols having 3 to 30 C atoms, these esters having no free hydroxyl groups;
(iii) esters of benzoic acid and linear or branched, saturated or unsaturated monoalkanols having 8 to 20 C atoms;
(iv) monoesters or diesters of alcohols having 3 to 30 C atoms and naphthalenemonocarboxylic or -dicarboxylic acids; especially naphthalenemonocarboxylic acid C₆-C₁₈ esters and naphthalenedicarboxylic acid di-C₆-C₁₈ esters;
(v) linear or branched, saturated or unsaturated di-C₆-C₁₈-alkyl ethers;
(vi) silicone oils;
(vii) 2-alkyl-1-alkanols of the formula (III) 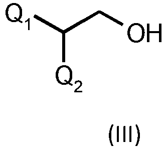 where
   Q₁ is a linear or branched alkyl radical having 6 to 24 C atoms and
   Q₂ is a linear or branched alkyl radical having 4 to 16 C atoms.

An oil phase or oil component encompasses the following groups of substances:
(i) linear or branched, saturated paraffins having 20 to 32 C atoms;
(ii) esters having at least 14 C atoms of linear or branched, saturated fatty acids having 8 to 24 C atoms and linear or branched, saturated or unsaturated mono-, di- or triols having 3 to 24 C atoms, these esters containing no free hydroxyl groups;
(iii) esters of benzoic acid and linear or branched, saturated monoalkanols having 10 to 18 C atoms;
(iv) 2,6-naphthalenedicarboxylic acid di-C6-C12 esters;
(v) linear or branched, saturated di-C6-C18-alkyl ethers, especially (straight-chain) di-C6-C12-alkyl ethers;
(vi) silicone oils from the group of the cyclotrisiloxanes, cyclopentasiloxanes, dimethylpolysiloxanes, diethylpolysiloxanes, methylphenylpolysiloxanes, diphenylpolysiloxanes and hybrid forms thereof;
(vii) 2-alkyl-1-alkanols having 12 to 32 C atoms of the formula (III)
   where
   Q₁ is a (preferably linear) alkyl radical having 6 to 18 C atoms and
   Q₂ is a (preferably linear) alkyl radical having 4 to 16 C atoms.

In a preferred way, said oil phase encompasses the following groups of substances:
(i) linear or branched, saturated paraffins having 20 to 32 C atoms such as isoeicosane or squalane;
(ii) esters having at least 16 C atoms of linear or branched, saturated fatty acids having 8 to 18 C atoms and linear or branched, saturated mono-, di- or triols having 3 to 18 C atoms, these esters containing no free hydroxyl groups;
(iii) esters of benzoic acid and linear or branched, saturated monoalkanols having 12 to 15 C atoms, especially C₁₂-₁₅-alkyl benzoates;
(iv) 2,6-naphthalenedicarboxylic acid di-C6-C10 esters, especially diethylhexyl 2,6-naphthalenedicarboxylate;
(v) straight-chain di-C₆-C₁₀-alkyl ethers; especially di-n-octyl ether (dicaprylyl ether);
(vi) silicone oils from the group undecamethylcyclotrisiloxane, cyclomethicone, decamethylcyclopentasiloxane, dimethylpolysiloxanes, diethylpolysiloxanes, methylphenylpolysiloxanes and diphenylpolysiloxanes;
(vii) 2-alkyl-1-alkanols having 12 to 32 C atoms of the formula (III)
   where
   Q₁ is a (preferably linear) alkyl radical having 6 to 18 C atoms and
   Q₂ is a (preferably linear) alkyl radical having 4 to 16 C atoms.

Particularly preferred components of type (i) in the oil phase are as follows: isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate, 2-ethylhexyl isostearate, isotridecyl isononanoate, 2-ethylhexyl cocoate, caprylic/capric triglyceride, and also synthetic, semisynthetic and natural mixtures of such esters, e.g. jojoba oil.

Fatty acid triglycerides (oil components of type (i) in the oil phase) may also be in the form of, or in the form of a constituent of, synthetic, semisynthetic and/or natural oils, examples being olive oil, sunflower oil, soya oil, peanut oil, rapeseed oil, almond oil, palm oil, coconut oil, palm kernel oil and mixtures thereof.

Particularly preferred oil components of type (vii) in the oil phase are as follows: 2-butyl-1-octanol, 2-hexyl-1-decanol, 2-octyl-1-dodecanol, 2-decyltetradecanol, 2-dodecyl-1-hexadecanol and 2-tetradecyl-1-octadecanol.

Particularly preferred oil components in the oil phase are mixtures comprising C₁₂-C₁₅-alkyl benzoate and 2-ethylhexyl isostearate, mixtures comprising C₁₂-C₁₅-alkyl benzoate and isotridecyl isononanoate, mixtures comprising C₁₂-C₁₅-alkyl benzoate, 2-ethylhexyl isostearate and isotridecyl isononanoate, mixtures comprising cyclomethicone and isotridecyl isononanoate, and mixtures comprising cyclomethicone and 2-ethylhexyl isostearate.

### INGREDIENTS HAVING CARE PROPERTIES

Preferred embodiments of the cosmetic and pharmacological preparations, especially dermatological preparations may further comprise ingredients having care properties, such as, for example, fatty alcohols having 6 to 30 C atoms. The fatty alcohols here can be saturated or unsaturated and linear or branched. Furthermore, these fatty alcohols can in some cases be part of the oil phase (III) if they correspond to the definition given there. Alcohols which can be employed are, for example, decanol, decenol, octanol, octenol, dodecanol, dodecenol, octadienol, decadienol, dodecadienol, oleyl alcohol, ricinoleyl alcohol, erucyl alcohol, stearyl alcohol, isostearyl alcohol, cetyl alcohol, lauryl alcohol, myristyl alcohol, arachidyl alcohol, caprylyl alcohol, capryl alcohol, linoleyl alcohol, linolenyl alcohol and behenyl alcohol, and also Guerbet alcohols thereof, such as, for example, 2-octyl-1-dodecanol, it being possible for the list to be extended virtually as desired by further alcohols of related structural chemistry. The fatty alcohols preferably originate from natural fatty acids, being conventionally prepared from the corresponding esters of the fatty acids by reduction. Fatty alcohol fractions which are formed by reduction from naturally occurring fats and fatty oils, such as beef tallow, peanut oil, colza oil, cottonseed oil, soya oil, sunflower oil, palm kernel oil, linseed oil, maize oil, castor oil, rapeseed oil, sesame oil, cacao butter and coconut fat, can further be employed.

Substances having care properties which advantageously can be employed in the cosmetic and pharmacological preparations, especially dermatological preparations can further include
- ceramides, where ceramides are understood as meaning N-acylsphingosins (fatty acid amides of sphingosin) or synthetic analogues of such lipids (so-called pseudo-ceramides), which significantly improve the water retention capacity of the stratum corneum.
- phospholipids, for example soya lecithin, egg lecithin and cephalins
- fatty acids
- phytosterols and phytosterol-containing fats or waxes
- vaseline, paraffin oils and silicone oils; the latter include, inter alia, dialkyl- and alkylarylsiloxanes, such as dimethylpolysiloxane and methylphenylpolysiloxane, and also alkoxylated and quaternised derivatives thereof.

### ALCOHOLS

The aqueous phase of the preparations optionally advantageously comprises alcohols, diols or polyols (lower alkyl), and ethers thereof, preferably ethanol, isopropanol, propylene glycol, 1,2-pentane diol,1,2-hexanediol, 1,2-octanediol, 1,2-decanediol, a mixture of 1,2-hexanediol and 1,2-octanediol, a mixture of 1,2-hexanediol and 1,2-decanediol, a mixture of 1,2-octanediol and 1,2-decanediol, a mixture of 1,2-hexanediol, 1,2-octanediol and 1,2-decanediol, glycerol, ethylene glycol-monoethyl or monobutyl ether, propylene glycol monomethyl, -monoethyl or monobutyl ether, diethylene glycol monomethyl or -monoethyl ether and analogous products, and also alcohols (lower alkyl), e.g. ethanol, 1,2-propanediol, glycerol, and, in particular, one or more thickeners which can advantageously be chosen from the group of silicon dioxide, aluminum silicates, polysaccharides and derivatives thereof, e.g. hyaluronic acid, xanthan gum, hydroxypropylmethylcellulose, particularly advantageously from the group of polyacrylates, preferably a polyacrylate from the group of so-called Carbomers, for example but not limited to, Carbopol® grades 980, 981, 1382, 2984, 5984, in each case individually or in combination.

### ANTI-INFLAMMATORY COMPOUNDS

Preferred embodiments of the cosmetic and pharmacological preparations, especially dermatological may also comprise active anti-inflammatory and/or redness- and/or itching-alleviating compounds (anti-irritants). All the active anti-inflammatory or redness- and/or itching-alleviating compounds which are suitable or usual for cosmetic, dermatological and pharmacological preparations can be used here. Active anti-inflammatory and redness- and/or itching-alleviating compounds which are advantageously employed are steroidal anti-inflammatory substances of the corticosteroid type, such as hydrocortisone, dexamethasone, dexamethasone phosphate, methylprednisolone or cortisone, it being possible for the list to be extended by addition of further steroidal anti-inflammatories. Non-steroidal anti-inflammatories can also be employed. Those to be mentioned here by way of example are oxicams, such as piroxicam or tenoxicam; salicylates, such as aspirin, Disalcid, Solprin or fendosal; acetic acid derivatives, such as diclofenac, fenclofenac, indomethacin, sulindac, tolmetin, or clindanac; fenamates, such as mefenamic, meclofenamic, flufenamic or niflumic; propionic acid derivatives, such as ibuprofen, naproxen, benoxaprofen or pyrazoles, such as phenylbutazone, oxyphenylbutazone, febrazone or azapropazone.

Alternatively, natural anti-inflammatory or redness- and/or itching-alleviating substances can be employed. Plant extracts, specific highly active plant extract fractions and highly pure active substances isolated from plant extracts can be employed. Extracts, fractions and active substances from chamomile, aloe vera, Commiphora species, Rubia species, willow, rose-bay willow-herb, oats, and also pure substances, such as, inter alia, bisabolol, apigenin 7-glucoside, boswellic acid, phytosterols, glycyrrhizic acid, glabridin or licochalcone A, are particularly preferred. The preparations can also comprise mixtures of two or more active anti-inflammatory compounds. Bisabolol, boswellic acid, and also extracts and isolated highly pure active compounds from oats and Echinacea are particularly preferred for use as anti-inflammatory and redness- and/or itching-alleviating substances, and alpha-bisabolol and extracts and isolated highly pure active compounds from oats are especially preferred.

The amount of anti-irritants (one or more compounds) in the preparations is preferably 0.0001% to 20% by weight, with particular preference 0.0001% to 10% by weight, in particular 0.001% to 5% by weight, based on the total weight of the preparation.

### MOISTURE REGULATORS

Preferred embodiments of the cosmetic and pharmacological preparations, especially dermatological preparations may advantageously also comprise moisture retention regulators. The following substances for example are used as moisture retention regulators (moisturizers): sodium lactate, urea, alcohols, sorbitol, glycerol, propylene glycol, aliphatic 1,2-diols with a C number of 5-10, collagen, elastin or hyaluronic acid, diacyl adipates, petrolatum, ectoin, urocanic acid, lecithin, panthenol, phytantriol, lycopene, algae extract, ceramides, cholesterol, glycolipids, chitosan, chondroitin sulphate, polyamino acids and polyamino sugars, lanolin, lanolin esters, amino acids, alpha-hydroxy acids (e.g. citric acid, lactic acid, malic acid) and derivatives thereof, sugars (e.g. inositol), alpha-hydroxy fatty acids, phytosterols, triterpene acids, such as betulinic acid or ursolic acid, algae extracts.

### PLANT EXTRACTS

Preferred embodiments of the the cosmetic and pharmacological preparations, especially dermatological preparations may advantageously also comprise plant extracts, which are conventionally prepared by extraction of the whole plant, but also in individual cases exclusively from blossom and/or leaves, wood, bark or roots of the plant. In respect of the plant extracts which can be used, reference is made in particular to the extracts which are listed in the table starting on page 44 of the 3rd edition of the Leitfaden zur Inhaltsstoffdeklaration kosmetischer Mittel [Manual of Declaration of the Constituents of Cosmetic Compositions], published by Industrieverband Körperpflegemittel und Waschmittel e.V. (IKW), Frankfurt. Extracts which are advantageous in particular are those from aloe, witch hazel, algae, oak bark, rose-bay willow-herb, stinging nettle, dead nettle, hops, chamomile, yarrow, arnica, calendula, burdock root, horsetail, hawthorn, linden blossom, almond, pine needle, horse chestnut, sandalwood, juniper, coconut, mango, apricot, orange, lemon, lime, grapefruit, apple, green tea, grapefruit pip, wheat, oats, barley, sage, thyme, wild thyme, rosemary, birch, mallow, lady's smock, willow bark, restharrow, colts-foot, hibiscus, ginseng and ginger root.

In this context, the extracts from aloe vera, chamomile, algae, rosemary, calendula, ginseng, cucumber, sage, stinging nettle, linden blossom, arnica and witch hazel are particularly preferred. Mixtures of two or more plant extracts can also be employed. Extraction agents which can be used for the preparation of plant extracts mentioned are, inter alia, water, alcohols and mixtures thereof. In this context, among the alcohols lower alcohols, such as ethanol and isopropanol, but also polyhydric alcohols, such as ethylene glycol, propylene glycol and butylene glycol, are preferred, and in particular both as the sole extraction agent and in mixtures with water. The plant extracts can be employed both in pure and in diluted form.

### PROTEIN HYDROLYSATES

Preferred embodiments of the cosmetic and pharmacological preparations, especially dermatological preparations may advantageously also comprise protein hydrolysates. Animal and/or plant protein hydrolysates can advantageously also be added to preferred embodiments of cosmetic and/or pharmaceutical, especially dermatologically active, preparations. Substances which are advantageous in this respect are, in particular, elastin, collagen, keratin, milk protein, soya protein, oat protein, pea protein, almond protein and wheat protein fractions or corresponding protein hydrolysates, and also condensation products thereof with fatty acids, and quaternised protein hydrolysates, the use of plant protein hydrolysates being preferred.

The aqueous phase of the preparations optionally comprises alcohols, diols or polyols (lower alkyl), and ethers thereof, preferably ethanol, isopropanol, propylene glycol, 1,2-hexanediol, 1,2-octanediol, 1,2-decanediol, a mixture of 1,2-hexanediol and 1,2-octanediol, a mixture of 1,2-hexanediol and 1,2-decanediol, a mixture of 1,2-octanediol and 1,2-decanediol, a mixture of 1,2-hexanediol, 1,2-octanediol and 1,2-decanediol, glycerol, ethylene glycol-monoethyl or monobutyl ether, propylene glycol monomethyl, -monoethyl or monobutyl ether, diethylene glycol monomethyl or -monoethyl ether and analogous products, and also alcohols (lower alkyl), e.g. ethanol, 1,2-propanediol, glycerol, and, in particular, one or more thickeners which can advantageously be chosen from the group of silicon dioxide, aluminum silicates, polysaccharides and derivatives thereof, e.g. hyaluronic acid, xanthan gum, hydroxypropylmethyl¬cellulose, particularly advantageously from the group of polyacrylates, preferably a poly¬acrylate from the group of so-called Carbopols, for example, Carbopol grades 980, 981, 1382, 2984, 5984, in each case individually or in combination.

### SACCHARIDES

Preferred embodiments of the cosmetic and/or pharmaceutical, especially dermatologically active preparations may advantageously also comprise mono- , di- and oligosaccharides, such as, for example, glucose, galactose, fructose, mannose, fruit sugars and lactose.

### COMPOSITIONS

The compositions can be selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the moisturizing and/or anti-ageing and/or wound healing promoting action thereof is more pronounced).

The formulations are typically in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C₆-C₃₂ fatty acid C₂-C₃₀ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

The formulations are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters)) or silicone oil, or a spray (e.g. pump spray or spray with propellant).

Auxiliary substances and additives can be included in quantities of 5 to 99 % b.w., preferably 10 to 80 % b.w., based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 % b.w., preferably 5 to 80 % b.w., based on the total weight of the preparation. It should be noted that the information on additives and their ranges for cosmetic compositions are also valid for pharmaceutical or dermatalogical formulations.

### EXAMPLES

### Example 1

**Sunscreen Spray, Expected SPF 30, UVA factor 27**

| **Part** | **Raw materials** | **INCI name** | **Amount % (w/w)** |
|---|---|---|---|
| A | Dracorin® GOC | Gylceryl Oleate Citrate, Caprylic/Capric Triglyceride | 2.00 |
| A | Neo Heliopan® 357 | Butyl Methoxydibenzoylmethane | 5.00 |
| A | Neo Heliopan® OS | Ethylhexyl Salicylate | 5.00 |
| A | Neo Heliopan® HMS | Homosalate | 10.00 |
| A | Neo Heliopan® 303 | Octocrylene | 6.00 |
| A | Eusolex® T-AVO | Titanium Dioxide, Silica | 3.00 |
| A | Dragoxat® 89 | Ethylhexyl Isononanoate | 5.00 |
| A | SymMollient® S | Cetaryl Nonanoate | 2.50 |
| A | Silsoft 034 | Caprylyl Methicone | 2.00 |
| A | Vitamin E Acetate | Tocopheryl Acetate | 0.50 |
| A | Silcare® Silicone 41M65 | C26-C28 Alkyl Dimethicone | 2.00 |
| A | Pemulen® TR-2 | AcrylatesC/10-30 Alkyl Acrylate Cross Polymer | 0.15 |
| A | EDETA® BD | Disodium EDTA | 0.10 |
| A | Dow Corning® 1503 Fluid | Dimethicone, Dimethiconol | 1.00 |
| A | Keltrol® CG BT | Xanthan Gum | 0.30 |
| B | Distilled Water | Water (Aqua) | Ad 100 |
| B | ExpertGel® 56 | Bis-Methoxy PEG-13 PEG-438/PPG-110 SMDI Copolymer | 0.50 |
| B | Neo Heliopan® AP | Disodium Phenyl Dibenzimidazole Tetrasulfonate | 4.60 |
| B | Triethanolamine 99% | Triethanolamine | 0.90 |
| B | Potassium Hydroxide | Potassium Hydroxide | Qs |
| B | Glycerin 99.5P | Glycerin | 3.00 |
| B | SymSol® PF-3 | Water(Aqua), Pentylene Glycol, Sodium Lauryl Sulfoacetate, Sodium Oleoyl Sarcosonate, Disodium Sulfoacetate | 3.00 |
| C | SymOcide® PS | Phenoxyethanol, Decylene Gylcol, 1,2-Hexanediol | 1.40 |
| D | Fragrance | Parfum | 0.20 |
| D | Dry Flo® TS | Tapioca Starch Polymethylsilsesquioxane | 1.50 |
| ***In Vitro SPF*** | | | ***33*** |
| ***UVA protection factor (Colipa recommended method)*** | | | ***27*** |

### Manufacturing Procedure for Example 1

- Prepare Part A without TiO2, Pemulen® and Keltrol® and heat to 50°C. Add TiO2, Keltrol® and Pemulen®, homogenise for 30s with an Ultra Turrax®.
- Prepare Part B by adding the ExpertGel® in water with stirring, once dissolved add TEA and KOH followed by Neo Heliopan® AP. Once fully dissolved add SymSol® PF3 and glycerine and stir until homogeneous.
- Add Part B to Part A and homogenise with the Ultra Turrax®. Then add Part C.
- At ambient temperature add the ingredients of Part D stepwise, then homogenise.
- The pH should be 7.5

### Example 2

**Water resistant sunspray, Expected SPF 50+, UVA factor 27**

| **Part** | **Raw materials** | **INCI name** | **Amount % (w/w)** |
|---|---|---|---|
| A | Dracorin® GOC | Gylceryl Oleate Citrate, Caprylic/Capric Triglyceride | 2.00 |
| A | Neo Heliopan® 357 | Butyl Methoxydibenzoylmethane | 5.00 |
| A | Neo Heliopan® E1000 | Isoamyl p-Methoxycinnamate | 2.00 |
| A | Neo Heliopan® OS | Ethylhexyl Salicylate | 5.00 |
| A | Neo Heliopan® HMS | Homosalate | 10.00 |
| A | Neo Heliopan® 303 | Octocrylene | 10.00 |
| A | Eusolex® T-AVO | Titanium Dioxide, Silica | 2.50 |
| A | Dragoxat® 89 | Ethylhexyl Isononanoate | 3.00 |
| A | SymMollient® S | Cetaryl Nonanoate | 2.00 |
| A | Wacker-Belsil® CDM 3526 VP | C26-C28 Alkyl Dimethicone | 1.00 |
| A | Silsoft ™ 034 | Caprylyl Methicone | 2.00 |
| A | Vitamin E Acetate | Tocopheryl Acetate | 0.50 |
| A | Silcare® Silicone 41M65 | C26-C28 Alkyl Dimethicone | 1.00 |
| A | Pemulen® TR-2 | AcrylatesC/10-30 Alkyl Acrylate Cross Polymer | 0.20 |
| A | EDETA® BD | Disodium EDTA | 0.10 |
| B | Distilled Water | Water (Aqua) | Ad 100 |
| C | ExpertGel® 56 | Bis-Methoxy PEG-13 PEG-438/PPG-110 SMDI Copolymer | 0.20 |
| C | Neo Heliopan® AP | Disodium Phenyl Dibenzimidazole Tetrasulfonate | 2.00 |
| C | Neo Heliopan® Hydro | Phenylbenzimidazole Sulfonic Acid | 2.00 |
| C | Sodium Hydroxide | Sodium Hydroxide | Qs |
| C | Biotive® L-Arginine | Arginine | 1.00 |
| C | SymSol® PF-3 | Water(Aqua), Pentylene Glycol, Sodium Lauryl Sulfoacetate, Sodium Oleoyl Sarcosonate, Disodium Sulfoacetate | 3.00 |
| C | Propylene Glycol | Propylene Glycol | 5.00 |
| C | SymOcide® PS | Phenoxyethanol, Decylene Gylcol, 1,2-Hexanediol | 1.25 |
| C | Dow Corning® 1503 Fluid | Dimethicone, Dimethiconol | 1.00 |
| D | Fragrance | Parfum | 0.40 |
| D | Dry Flo® TS | Tapioca Starch Polymethylsilsesquioxane | 1.50 |
| ***In Vitro SPF*** | | | 116 |
| ***UVA protection factor (Colipa recommended method)*** | | | 27 |

### Manufacturing Procedure for Example 2

- Prepare Part A without Pemulen® and heat to 60°C. Add Pemulen®, homogenise for 30s with an Ultra Turrax®.
- Prepare Part B by adding the ExpertGel® in water with stirring, once dissolved add NaOH and arginine followed by Neo Heliopan® Hydro and Neo Heliopan® AP. Once fully dissolved add SymSol® PF3 and propylene glycol and stir until clear and homogeneous.
- Add Part B to Part A without stirring and homogenise with the Ultra Turrax®. Then add Part C.
- At ambient temperature add the ingredients of Part D stepwise, then homogenise.
- The pH should be 6.5

### Example 3

**Sun Gel, expected SPF 30, UVA Protection factor 19**

| **Part** | **Raw materials** | **INCI name** | **Amount % (w/w)** |
|---|---|---|---|
| A | Water | Water (Aqua) | 42,00 |
| B | Potassium Hydroxide (10% aq) | Potassium Hydroxide | 28.8 |
| B | Biotive® L-Arginine | Arginine | 1.00 |
| B | Neo Heliopan® AP | Disodium Phenyl Dibenzimidazole Tetrasulfonate | 6.00 |
| B | Neo Heliopan® Hydro | Phenylbenzimidazole Sulfonic Acid | 7.00 |
| B | Dragosine® | Carnosine | 0.20 |
| B | Hydrolite® 5 | Pentylene Glycol | 4.00 |
| B | Glycerine 99.5 P | Glycerin | 2.00 |
| B | EDETA® B fl. | Tetrasodium EDTA | 0.10 |
| B | Cesmatic 4W | Hydroxypropyl Guar | 1.00 |
| C | Dermacryl® AQF | Acrylates Copolymer | 1.00 |
| D | SymMollient® W/S | Trideceth-9, PEG-5 Isononanoate, Water (Aqua) | 1.7 |
| D | Fragrance | Parfum | 0.20 |
| D | Ethanol 96% | Ethanol | 10.00 |
| ***In Vitro SPF*** | | | ***31*** |
| ***UVA protection factor (Colipa recommended method)*** | | | ***19*** |

### Manufacturing Procedure for Example 3

Mix components of Part A at ambient temperature until a clear solution is obtained. Add Part B to Part A, stir until homogeneous, the add Part C and stir until homogeneous. Prepare Part D and add to Parts A/B/C with stirring and homogenise. The pH should be 7.5.

### Example 4

**Sunscreen lotion (O/W), expected SPF 50**

| **Part** | **Raw Materials** | **INCI Name** | **Amount % (wt.)** |
|---|---|---|---|
| A | Emulsiphos | Potassium Cetyl Phosphate, Hydrogenated Palm Glycerides | 2.00 |
| A | Lanette® 16 | Cetyl Alcohol | 1.00 |
| A | Neo Heliopan® 357 | Butyl Methoxydibenzolymethane | 3.00 |
| A | Neo Heliopan® OS | Ethylhexyl Salicylate | 5.00 |
| A | Neo Heliopan® 303 | Octocrylene | 8.00 |
| A | Neo Heliopan® HMS | Homosalate | 5.00 |
| A | Dragoxat® 89 | Ethylhexyl Isononanoate | 5.00 |
| A | KF-995 | Cyclopentasiloxane | 2.00 |
| A | EDETA® BD | Disodium EDTA | 0.10 |
| A | Vitamin E Acetate | Tocopheryl Acetete | 0.50 |
| A | Titanium Dioxide (nano) | Titanium Dioxide | 4.00 |
| A | Keltrol® T | Xanthan Gum | 0.50 |
| B | Water | Aqua (Water) | 51.65 |
| B | Troxerutin | Troxerutin | 0.30 |
| B | Neo Heliopan® AP, 15% neutralisised with Arginine | Disodium Phenyl Dibenzimidazole Tetrasulfonate | 6.70 |
| B | Glycerin 99% | Glycerin | 3.00 |
| B | SymOcide® PS | Phenoxyethanol, Decylene Gylcol, 1,2-Hexanediol | 1.25 |
| B | Lanette® E | Sodium Cetearyl Sulfate | 0.75 |
| C | Pafüm | Parfum | 0.20 |
| C | α-Bisabolol | Bisabolol | 0.10 |
| ***In Vitro SPF*** | | | ***55*** |
| ***UVA protection factor (Colipa recommended method)*** | | | ***17*** |

### Manufacturing Procedure for Example 4

Part A: Mix the ingredients without Keltrol® und Titanium Dioxide and heat to 85°C and homogenise for a short time.
Part B: Mix the ingredients with stirring and heat to 85°C until dissolved. Add Part B to A without stirring then stir the mixture and allow to cool to 60°C. Then homogenise.
Part C: At ambient temperature add the components of Part C to Part A/B with stirring until homogeneous, then homogenise.

### Example 5

**Sunscreen fluid lotion (O/W), expected SPF 50 Plus**

| **Part** | **Raw Materials** | **INCI Name** | **Amount % (wt.)** |
|---|---|---|---|
| A | Emulsiphos | Potassium Cetyl Phosphate, Hydrogenated Palm Glycerides | 0.80 |
| A | Dracorin® 100 SEP | Glyceryl Stearate, PEG-100 Stearate | 1.50 |
| A | Neo Heliopan® 357 | Butyl Methoxydibenzolymethane | 5.00 |
| A | Neo Heliopan® OS | Ethylhexyl Salicylate | 5.00 |
| A | Neo Heliopan® 303 | Octocrylene | 10.00 |
| A | Neo Heliopan® HMS | Homosalate | 10.00 |
| A | Neo Heliopan® E1000 | Isoamyl p-Methoxycinnamate | 3.00 |
| A | Titanium Dioxide (nano) | Titanium Dioxide | 2.50 |
| A | Isoadipate, 660014 | Diisopropyl Adipate | 2.50 |
| A | Silsoft ™ 034 | Caprylyl Methicone | 1.00 |
| A | Dow Corning® Wax 2503 | Stearyl Dimethicone | 1.00 |
| A | Dow Corning® EL 7040 Hydro Elastomer Blend | Caprylyl Methicone (and) PEG-12 Dimethicone/PPG-20 Crosspolymer | 2.00 |
| A | Prisorine® 3505 | Isostearic Acid | 1.00 |
| A | EDETA® BD | Disodium EDTA | 0.10 |
| A | Copherol® 1250 | Tocopheryl Acetete | 0.50 |
| A | Keltrol® CG SFT | Xanthan Gum | 0.15 |
| A | Pemulen® TR 2 | Acrylates/C10-30 Alkyl Acrylate Crosspolymer | 0.15 |
| B | Water dem | Aqua (Water) | 34.60 |
| B | Neo Heliopan® Hydro | Phenylbenzimidazole Sulfonic Acid | 3.00 |
| B | Neo Heliopan® AP | Disodium Phenyl Dibenzimidazole Tetrasulfonate | 1.00 |
| B | Biotive® L-Arginine | Arginine | 1.20 |
| B | Glycerin 99% | Glycerin | 1.00 |
| B | Propylenglykol | Propylene Glycol | 5.00 |
| B | NaOH 10% aq. | Sodium Hydroxide | 3.00 |
| B | SymSave® H | Hydroxyacetophenone | 0.50 |
| C | Symdiol® 68 | 1,2-Hexanediol, Caprylyl Glycol | 0.50 |
| C | Dow Corning® 9801 Cosmetic Powder | Dimethicone / Vinyl Dimethicone Crosspolymer, Silica | 2.00 |
| C | Tapioca pure | Tapioca Starch | 2.00 |
| ***In Vitro SPF*** | | | ***65*** |
| ***UVA protection factor (Colipa recommended method)*** | | | ***22*** |

### Manufacturing Procedure for Example 5

Part A: Mix the components without Keltrol® and TiO2 and heat to approx 85°C. Add Keltrol® and TiO2 and homogenize for a short time, approx 0,5 min. with an Ultra Turrax® T25.
Part B: Mix the components while heating up to 80°C until a clear solution is obtained.
   Add the water Part B without stirring to the warm oil Part A. Stir to cool down to 60°C, then start homogenizing with an Ultra Turrax®. Cool down while stirring.
Part C: Add ingredients of Part C while stirring to Part A/B at ambient temperature.

Homogenize with an Ultra Turrax® for a short time.

### Example 6

**Sunscreen balm (O/W), expected SPF 50**

| **Part** | **Raw Materials** | **INCI Name** | **Amount % (wt.)** |
|---|---|---|---|
| A | Water | Aqua | 40.10 |
| A | Neo Heliopan® Hydro | Phenylbenzimidazole Sulfonic Acid | 3.00 |
| A | Neo Heliopan ®AP | Disodium Phenyl Dibenzimidazole Tetrasulfonate | 2.00 |
| A | Biotive® L-Arginine | Arginine | 1.50 |
| A | Carbopol® Ultrez 20 | Acrylates/C10-30 Alkyl Acrylate Crosspolymer | 0.70 |
| A | Glycerin 99% | Glycerin | 3.00 |
| A | Keltrol® SFT | Xanthan Gum | 0.10 |
| A | SymSave® H | Hydroxyacetophenone | 0.50 |
| A | NaOH 10% | Sodium Hydroxide | 6.00 |
| B | Neo Heliopan® HMS | Homosalate | 10.00 |
| B | Neo Heliopan® 303 | Octocrylene | 10.00 |
| B | Neo Heliopan® OS | Ethylhexyl Salicylate | 5.00 |
| B | Neo Heliopan ®357 | Butyl Methoxydibenzoylmethane | 5.00 |
| B | Neo Heliopan® E1000 | Isoamyl p-Methoxycinnamate | 2.00 |
| B | Titanium Dioxide (nano) | Titanium Dioxide | 2.00 |
| B | Silcare® Silicone 41M65 | Stearyl Dimethicone | 1.00 |
| B | Floraesters® K100 | Hydrolyzed Jojoba Esters (and) Jojoba Esters (and) Water (Aqua) | 1.00 |
| B | Tocopherylacetat | Tococpheryl Acetate | 0.50 |
| B | Wacker-Belsil® CDM 3526 VP | C26-28 Alkyl Dimethicone | 1.00 |
| B | EDETA® BD | Disodium EDTA | 0.10 |
| C | Ethanol 96% | Alcohol | 3.00 |
| C | SymDiol® 68 | 1,2 Hexanediol, Caprylylglycol | 0.50 |
| C | Dow Corning® 9701 Cosmetic Powder | Dimethicone / Vinyl Dimethicone Crosspolymer, Silica | 2.00 |
| ***In Vitro SPF*** | | | ***55*** |
| ***UVA protection factor (Colipa recommended method)*** | | | ***17*** |

### Manufacturing Procedure for Example 6

Part A: Mix Part A without SymSave®, Keltrol® and NaOH with an Ultra Turrax. Start stirring with a vane stirrer, then add Keltrol® and SymSave® and stir until a homogeneous turbid solution is obtained. Add NaOH while stirring thoroughly until solution becomes a clear gel.
Part B: Mix the ingredients without TiO2 while heating up to 60°C until a clear solution is obtained. Add TiO2 and homogenize for a short time.
   Add the Part B slowly with stirring to the water Part A. Cool down while stirring.
Part C: Add ingredients of Part C while stirring to Part A/B at ambient temperature.

Start homogenizing with an Ultra Turrax® until a homogenous balm is obtained.

### Example 7

**Anti-aging Sunscreen lotion (O/W), expected SPF 30**

| **Part** | **Raw Materials** | **INCI Name** | **Amount % (wt.)** |
|---|---|---|---|
| A | Emulsiphos® | Potassium Cetyl Phosphate, Hydrogenated Palm Glycerides | 3.00 |
| A | Lanette® 16 | Cetyl Alcohol | 1.00 |
| A | Neo Heliopan® 357 | Butyl Methoxydibenzolymethane | 3.00 |
| A | Neo Heliopan® OS | Ethylhexyl Salicylate | 5.00 |
| A | Neo Heliopan® 303 | Octocrylene | 8.00 |
| A | Neo Heliopan® HMS | Homosalate | 10.00 |
| A | Titanium Dioxide (nano) | Titanium Dioxide | 2.00 |
| A | SymHelios® 1031 | Benzylidene Dimethoxydimethylindanone | 0.50 |
| A | Silsoft™ 034 | Caprylyl Methicone | 3.00 |
| A | Dragoxat® 89 | Ethylhexyl Isononanoate | 3.00 |
| A | SymSitive ®1609 | Pentylene Glycol, 4-t-Butylcylcohexanol | 1.00 |
| A | Antaron® WP 660 | Tricontanyl PVP | 1.00 |
| A | EDETA® BD | Disodium EDTA | 0.10 |
| A | Copherol® 1250 | Tocopheryl Acetete | 0.50 |
| A | Keltrol® T | Xanthan Gum | 0.40 |
| A | Wacker-Belsil® CDM 3526 VP | C26 - 28 Alkyl Dimethicone | 1.00 |
| A | Hydrolite®-5 | Pentylene glycol | 4.25 |
| A | SymMollient® S | Cetearyl Nonanoate | 1.00 |
| B | Water | Aqua (Water) | 40.55 |
| B | Neo Heliopan® Hydro | Phenylbenzimidazole Sulfonic Acid | 1.00 |
| B | Neo Heliopan® AP | Disodium Phenyl Dibenzimidazole Tetrasulfonate | 1.00 |
| B | Biotive® L-Arginine | Arginine | 1.00 |
| B | Glycerin 99% | Glycerin | 2.00 |
| B | Lanette® E | Sodium Cetearyl Sulfate | 0.50 |
| B | NaOH 10% aq. | Sodium Hydroxide | 0.60 |
| C | SymSave® H | Hydroxyacetophenone | 0.50 |
| C | Symdiol® 68 | 1,2-Hexanediol, Caprylyl Glycol | 0.50 |
| C | Dow Corning® 9701 Cosmetic Powder | Dimethicone / Vinyl Dimethicone Crosspolymer, Silica | 0.50 |
| C | Parfüm | Parfum | 0.20 |
| C | Dragosantol® 100 | Bisabalol | 0.10 |
| C | SymGlucan® | Aqua, Glycerin, Beta-Glucan, 1,2-Hexanediol, Caprylyl Glycol | 1.00 |
| C | Orgasol® Caresse | Nylon 6/12 | 2.00 |
| ***In Vitro SPF*** | | | ***45*** |
| ***UVA protection factor (Colipa recommended method)*** | | | ***13*** |

### Manufacturing Procedure for Example 7

Part A: Mix the components without TiO2 and Keltrol® T to approx. 85°C, then add TiO2 and Keltrol® T. Homogenize for a short time with an Ultra Turrax®.
Part B: Mix the components and heat up to approx. 80°C until dissolved. Add Part B to Part A while stirring. Cool down while stirring to 60°C and homogenize with an Ultra Turrax®. Then cool down to ambient temperature while stirring.
Part C: Add all ingredients step by step and stir until homogeneous. Check the pH value. The pH value must be approx. 6.4. If the pH value is correct homogenize with an Ultra Tur-rax®.

### Example 8

**Low viscosity sunscreen lotion (O/W), expected SPF 50+**

| **Part** | **Raw Materials** | **INCI Name** | **Amount % (wt.)** |
|---|---|---|---|
| A | Emulsiphos® | Potassium Cetyl Phosphate, Hydrogenated Palm Glycerides | 0.75 |
| A | Dracorin® 100 SEP | Glyceryl Stearate, PEG-100 Stearate | 1.00 |
| A | Neo Heliopan® 357 | Butyl Methoxydibenzolymethane | 5.00 |
| A | Neo Heliopan® OS | Ethylhexyl Salicylate | 5.00 |
| A | Neo Heliopan® 303 | Octocrylene | 10.00 |
| A | Neo Heliopan® HMS | Homosalate | 10.00 |
| A | Neo Heliopan® E1000 | Isoamyl p-Methoxycinnamate | 3.00 |
| A | Uvasorb® HEB | Diethylhexyl Butamindo Triazone | 2.00 |
| A | Isoadipate, 660014 | Diisopropyl Adipate | 3.00 |
| A | Dow Corning® Wax 2503 | Stearyl Dimethicone | 2.00 |
| A | Dow Corning® EL 7040 Hydro Elastomer Blend | Caprylyl Methicone (and) PEG-12 Dimethicone/PPG-20 Crosspolymer | 3.00 |
| A | EDETA® BD | Disodium EDTA | 0.10 |
| A | Copherol® 1250 | Tocopheryl Acetete | 0.50 |
| A | Keltrol® CG SFT | Xanthan Gum | 0.15 |
| A | Pemulen® TR 2 | Acrylates/C10-30 Alkyl Acrylate Crosspolymer | 0.15 |
| B | Water | Aqua (Water) | 34.90 |
| B | Neo Heliopan® Hydro | Phenylbenzimidazole Sulfonic Acid | 2.00 |
| B | Neo Heliopan® AP | Disodium Phenyl Dibenzimidazole Tetrasulfonate | 2.20 |
| B | Biotive® L-Arginine | Arginine | 1.00 |
| B | Glycerin 99% | Glycerin | 2.00 |
| B | Propylenglycol | Propylene Glycol | 3.00 |
| B | NaOH 10% aq. | Sodium Hydroxide | 3.75 |
| B | Hydrolite® 5, 616751 | Pentylene Glycol | 1.00 |
| B | Phenoxyethanol | Phenoxyethanol | 0.50 |
| C | Symdiol® 68, 108580 | 1,2-Hexanediol, Caprylyl Glycol | 1.00 |
| D | Dow Corning® 9701 Cosmetic Powder | Dimethicone / Vinyl Dimethicone Crosspolymer, Silica | 1.00 |
| D | Orgasol® 4000 EXD NAT COS Caresse | Nylon 6/12 | 2.00 |
| ***In Vitro SPF*** | | | ***77*** |
| ***UVA protection factor (Colipa recommended method)*** | | | ***23*** |

### Manufacturing Procedure for Example 8

Part A: Mix ingredients without Keltrol® and Pemulen®, and heat up to approx. 85°C. When all ingredients are dissolved add Keltrol® and Pemulen® and homogenize with an Ultra Turrax® for a short time.
Part B: Mix ingredients and heat up to approx 80°C. Add Part B to Part A and homoge-nizing with an Ultra Turrax® (13000rpm/1minutes per 100g emulsion). Cool down to ambient temperature while stirring.
Part C: Add to Part A/B with stiring until homogeneous.
Part D: Add to Parts A/B/C while stirring. Homogenize with an Ultra Turrax (13000rpm/ 1minutes per 100g emulsion).

### Example 9

**Low oil content Sunscreen milk (O/W), in-vitro SPF 25**

| **Part** | **Raw Materials** | **INCI Name** | **Amount % (wt.)** |
|---|---|---|---|
| A | Tegin M | Glyceryl Stearate | 2.50 |
| A | Tagat S | PEG-30 Glyceryl Stearate | 1.95 |
| A | Lanette O | Cetearyl Alcohol | 2.20 |
| A | Copherol 1250 | Tocopheryl Acetate | 0.50 |
| A | Phenonip | Phenoxyethanol (and) methylparaben (and) Butylparaben (and) ethyl-paraben (and) Propylparaben | 0.15 |
| A | Neo Heliopan® AV | Ethylhexyl Methoxycinnamate | 5.00 |
| A | Neo Heliopan® 303 | Octocrylene | 5.00 |
| A | Neo Heliopan® 357 | Butyl Methoxydibenzolymethane | 2.00 |
| A | EDETA BD | Disodium EDETA | 0.10 |
| B | Water, dist. | Water (Aqua) | Ad 100 |
| B | Neo Heliopan® Hydro | Phenylbenzimidazole Sulfonic Acid, | 3.30 |
| B | Neo Heliopan® AP | Disodium Phenyl Dibenzimidazole Tetrasulfonate | 2.20 |
| B | Troxerutin | Troxerutin | 0.5 |
| B | 1,2-Propylene glycol | Propylene Glycol | 2.00 |
| B | Phenonip® | Phenoxyethanol (and) Methylparaben (and) Butylparaben (and) Ethylparaben (and) Propylparaben | 0.30 |
| B | NaOH, 10% strength | Sodium Hydroxide | 2.2 |
| C | Water, dist. | Water (Aqua) | 19.00 |
| C | Carbopol® ETD 2050 | Carbomer | 0.40 |
| C | NaOH, 10% strength | Sodium Hydroxide | qs |
| D | Perfume oil | Parfum (Fragrance) | 0.30 |

### Manufacturing procedure for Example 9

Part A: Heat to 80-85°C.
Part B: Heat to 80-85°C, Add part B to part A with stirring.
Part C: Disperse Carbopol® into the water and neutralise with NaOH, with stirring. Add part C at about 60°C with stirring. Allow to cool to room temperature.
Part D: Add and stir.

### Example 10

**Sunscreen spray in-vitro SPF 20**

| **Part** | **Raw Materials** | **INCI Name** | **Amount % (wt.)** |
|---|---|---|---|
| A | Water, demin. | Water (Aqua) | 62.1 |
| A | Glycerol, 99% | Glycerin | 4.00 |
| A | Hydrolite® 5 | 1,2-Pentylene Glycol | 5.00 |
| A | D-Panthenol | Panthenol | 0.50 |
| A | Lara Care® A-200 | Galactoarabinan | 0.25 |
| B | Baysilone® oil M 10 | Dimethicone | 1.00 |
| B | Edeta® BD | Disodium EDTA | 0.10 |
| B | Copherol ®1250 | Tocopheryl Acetate | 0.50 |
| B | Cetiol ®OE | Dicaprylyl Ether | 3.00 |
| B | Neo Heliopan® HMS | Homosalate | 5.00 |
| B | Neo Heliopan® AV | Ethylhexyl Methoxycinnamate | 6.00 |
| B | Neo Heliopan® 357 | Butyl Methoxydibenzoylmethane | 1.00 |
| B | Alpha-Bisabolol | Bisabolol | 0.10 |
| B | Pemulen® TR-2 | Acrylates/C10-30 Alkyl Acrylate Crosspolymer | 0.25 |
| C | Phenoxyethanol | Phenoxyethanol | 0.70 |
| C | Solbrol® M | Methylparaben | 0.20 |
| C | Solbrol® P | Propylparaben | 0.10 |
| C | Neo Heliopan® Hydro | Phenylbenzimidazole Sulfonic Acid | 2.00 |
| C | Neo Heliopan® AP | Disodium Phenyl Dibenzimidazole Tetrasulfonate | 1.50 |
| C | Troxerutin | Troxerutin | 0.5 |
| C | Arginine | Arginine | qs |
| D | Perfume oil | Fragrance (Parfum) | 0.20 |

### Manufacturing procedure for Example 10

Part A: Dissolve Lara Care® A-200 into the other constituents of part A with stirring.
Part B: Weigh in all raw materials (without Pemulen®) and dissolve the crystalline substances with heating. Disperse Pemulen® therein. Add part B to part A then homogenise for 1 minute.
Part C: Stirr in the ingredients until all have dissolved and add part C+D then homogenise again for 1-2 minutes using the Ultra Turrax. The pH of the formulation is 6.2

### Example 11

**Water resistant Broad Spectrum O/W emulsion in-vitro SPF 50+**

| **Part** | **Ingredients** | **INCI** | **Amount % (wt.)** |
|---|---|---|---|
| A | Emulsiphos® | Potassium Cetyl Phosphate, Hydrogenated Palm Glycerides | 3.50 |
| A | Lanette® O | Cetearyl alcohol | 1.00 |
| A | Neo Heliopan® HMS | Homosalate | 5.00 |
| A | Neo Heliopan® 303 | Octocrylene | 10.00 |
| A | Neo Heliopan® OS | Ethylhexyl Salicylate | 5.00 |
| A | Neo Heliopan® 357 | Butyl Methoxydibenzoylmethane | 5.00 |
| A | Eusolex® T2000 | Titanium Dioxide, Alumina, Simethicone | 5.00 |
| A | Abil Wax® 9801 | Cetyl Dimethicone | 1.00 |
| A | Silcare® Silicone 41M65 | Stearyl Dimethicone | 1.00 |
| A | Baysilone® oil PK 20 | Phenyl Trimethicone | 2.00 |
| A | Isoadipat | Diisopropyladipate | 2.00 |
| A | Tocopherylacetat | Tocopheryl Acetate | 0.50 |
| A | Antaron® V216 | VP/Hexadecene Copolymer | 0.50 |
| A | EDETA® BD | Disodium EDTA | 0.10 |
| A | Keltrol® T | Xanthan Gum | 0.50 |
| B | Water dem | Water (Aqua) | Ad 100 |
| B | Troxerutin | Troxerutin | 1.0 |
| B | Neo Heliopan® AP | Disodium Phenyl Dibenzimidazole Tetrasulfonate | 2.00 |
| B | SymSave® H | Hydroxyacetophenone | 0.50 |
| B | Arginine | Arginine | 2.20 |
| B | Lara Care® A-200 | Galactoarabinan | 0.25 |
| B | Hydrolite® 5 | Pentylene Glycol | 3.00 |
| C | Fragrance | Fragrance (parfum) | 0.30 |

### Manufacturing procedure for Example 11

Part A: Heat all components except for the Xanthan Gum and TiO2 to 85°C. Then add Xanthan Gum and TiO2 and homogenise.
Part B: Heat all components to 85°C and add to Part A with stirring, stir to room temperature.
Part C: Add Part C to Parts A and B and homogenise.

### Example 12

**Clear Hair Shampoo with UV-Protection**

| **Part** | **Ingredients** | **INCI-Name** | **Amount% (wt.)** |
|---|---|---|---|
| A | Demineralised Water | Water (Aqua) | Ad 100 |
| A | Merquat® 550 | Polyquaterium-7 | 0.50 |
| B | Neo Heliopan® AP | Disodium Phenyl Dibenzimidazole Tetrasulfonate | 1.00 |
| B | Troxerutin | Troxerutin | 0.3 |
| B | Amino Methyl Propanol | Amino Methyl Ppropanol | 0.6 |
| C | Genapol®LRO Liquid | Sodium Laureth Sulfate | 30.00 |
| C | Tego® Betain F 50 | Cocoamidopropyl Betaine | 5.00 |
| C | Antil®141 | Propylene Glycol, PEG-55 Propylene Glycol Dioleate | 0.80 |
| C | SymSave® H | Hydroxyacetophenone | 0.80 |
| C | D-Panthenol 75 L | Panthenol | 1.00 |
| C | Extrapone® Lime | Propylene Glycol, Water (Aqua), Citrus Aurantifolia (Lime) Juice | 1.00 |
| C | Sodium Chloride | Sodium Chloride | 0.70 |
| C | Perfume | Fragrance | 0.40 |

### Manufacturing procedure for example 12

Part A: Dissolve Merquat in water with stirring
Part B: Add Neo Heliopan Hydro and neutralise with Amino Methyl Propanol, dissolve until a clear solution has formed.
Part C: Add the ingredients to parts A/B as listed and stir until a uniform solution has formed. The viscosity can be adjusted by the amount of sodium chloride. The pH of the resulting formulation was in the range of 5.2 - 5.5

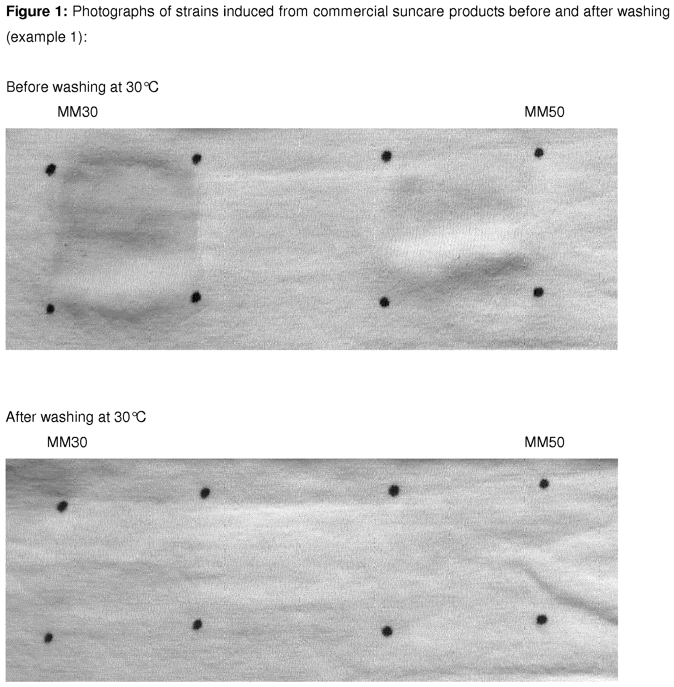

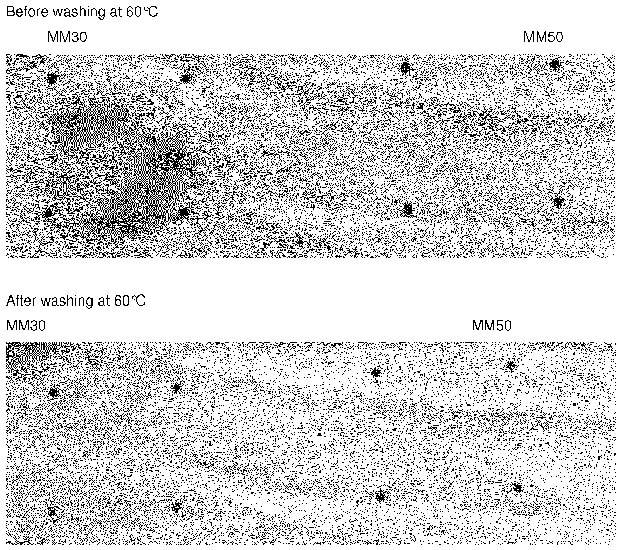

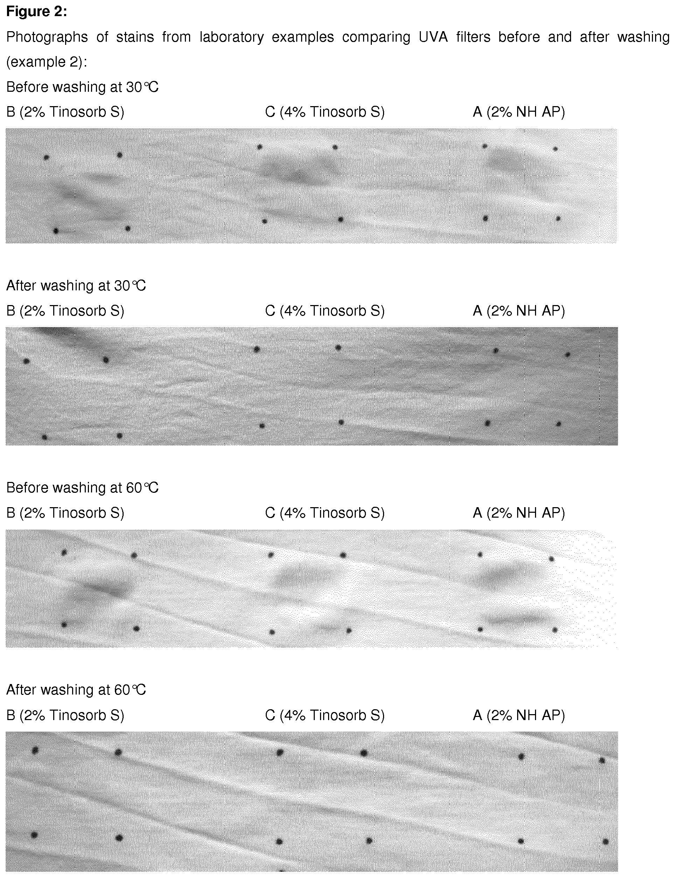

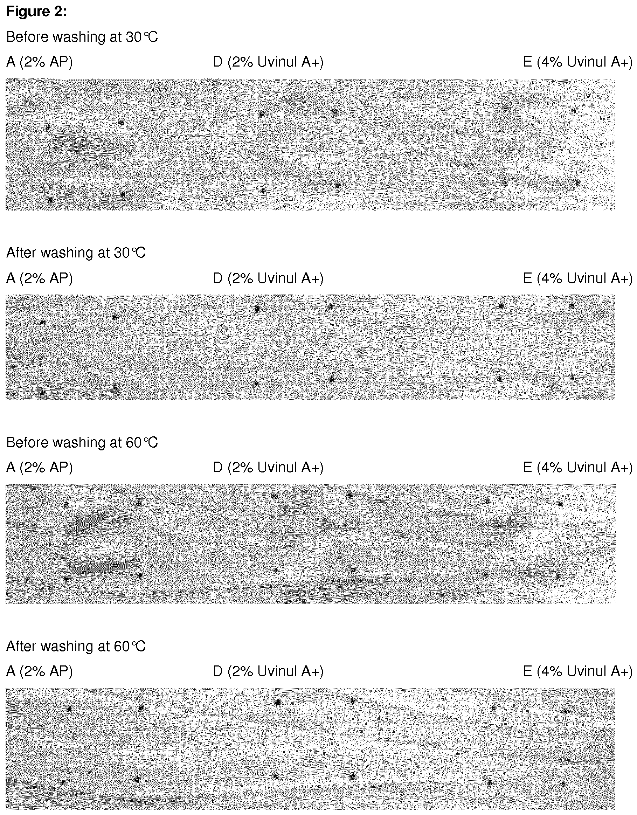

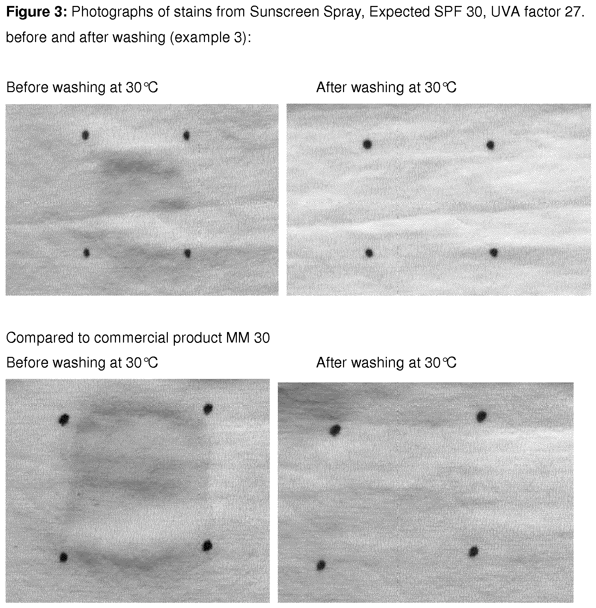

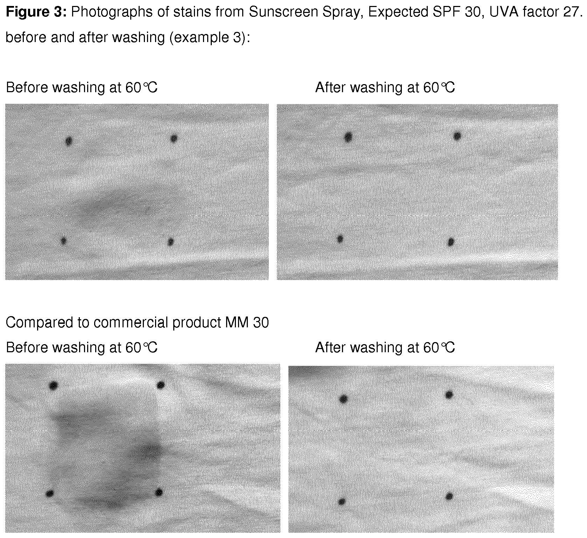

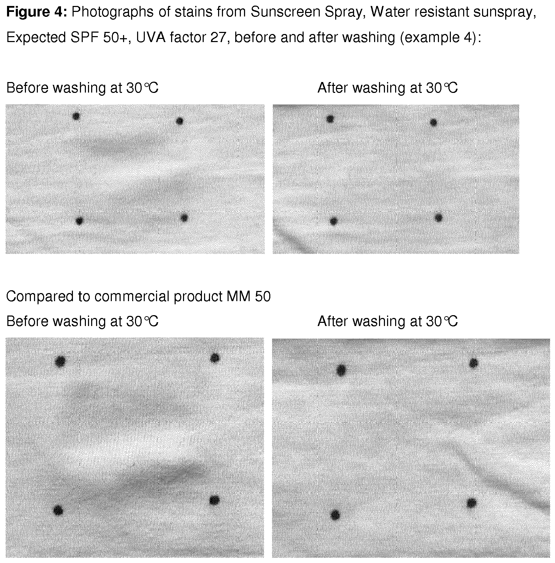

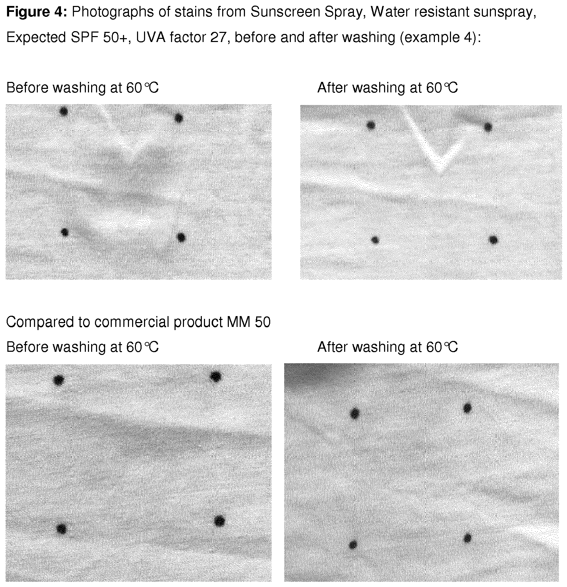

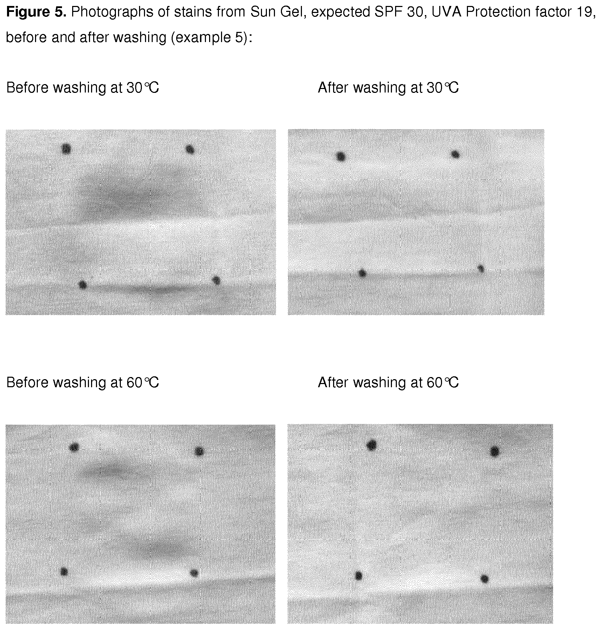

## Claims

1. An aqueous mixture comprising
(a) disodium phenyl benzimidazole tetrasulfonic acid and/or its salts, and
(b) 1-(4-Methoxyphenyl)-3-(4-tert-butylphenyl)propane-1,3-dione (Avobenzone), and
(c) 2,6- diethylhexyl naphthalate, and
(d) at least one additional UV absorbing substance selected from the group consisting of:
- 2-ethylhexyl 2-cyano-3,3-diphenylacrylate
- 2-ethylhexyl p-methoxycinnamate
- isoamyl p-methoxycinnamate
- 2,4,6-trianilino(p-carbo-2'-ethylhexyl-1'-oxy)-1,3,5-triazine
- 3-(4'-methylbenzylidene)-d,l-camphor
- Benzophenone-4
- menthyl anthranilate
- micro fine zinc oxide, and
(e) water
for use as a medicament for protecting human skin from the harmful effects of ultraviolet radiation of from 280 to 400 nm

## Patentansprüche

1. Eine wässrige Mischung, umfassend
(a) Dinatriumphenylbenzimidazol-Tetrasulfonsäure und/oder deren Salze, und
(b) 1-(4-Methoxyphenyl)-3-(4-tert-Butylphenyl)propan-1,3-dion (Avobenzon) und
(c) Diethylhexylnaphthalat, und
(d) mindestens eine zusätzliche UV-absorbierende Substanz, ausgewählt aus der Gruppe bestehend aus:
- 2,6-Ethylhexyl-2-Cyano-3,3-diphenylacrylat
- 2-Ethylhexyl-p-Methoxycinnamat
- Isoamyl-p-Methoxycinnamat
- 2,4,6-Trianilino(p-Carbo-2'-ethylhexyl-1'-oxy)-1,3,5-triazin
- 3-(4'-Methylbenzyliden)-d,l-Kampfer
- Benzophenon-4
- Menthyl-Anthranilat
- mikrofeines Zinkoxid, und
(e) Wasser
zur Verwendung als Medikament zum Schutz der menschlichen Haut vor den schädlichen Auswirkungen der ultravioletten Strahlung im Bereich von 280 bis 400 nm.

## Revendications

1. Un mélange aqueux comprenant
(a) l'acide phénylbenzimidazole tétrasulfonique disodique et/ou ses sels, et
(b) 1-(4-méthoxyphényl)-3-(4-tert-butylphényl)propane-1,3-dione (avobenzone) ; et
(c) le naphtalate de diéthylhexyle, et
(d) au moins une substance supplémentaire absorbant les UV, choisie dans le groupe constitué par
- 2,6-éthylhexyl-2-cyano-3,3-diphénylacrylate
- 2-éthylhexyl-p-méthoxycinnamate
- isoamyl p-méthoxycinnamate
- 2,4,6-Trianilino(p-Carbo-2'-éthylhexyl-1'-oxy)-1,3,5-triazine
- 3-(4'-méthylbenzylidène)-d,l-camphre
- benzophénone-4
- anthranilate de menthyle
- l'oxyde de zinc microfin, et
(e) eau
pour une utilisation comme médicament destiné à protéger la peau humaine des effets nocifs des rayons ultraviolets dans la gamme de 280 à 400 nm.
